# EUROPEAN PATENT SPECIFICATION

(11) **EP 0 885 861 B1**
(45) Date of publication and mention of the grant of the patent: **31.07.2002**
(21) Application number: 96938479.1
(22) Date of filing: 15.11.1996
(51) Int. Cl.: C07C 13/28, C07C 22/00, C07C 25/24, C07C 43/192, C07C 43/225, C07C 255/46, C07C 255/50, C09K 19/30, G02F 1/13

(54) **LIQUID-CRYSTAL COMPOUNDS BEARING VINYL LINKAGES AND LIQUID-CRYSTAL COMPOSITION**
FLÜSSIGKRISTALLINE VERBINDUNGEN, DIE VINYLBINDUNGEN ENTHALTEN UND FLÜSSIGKRISTALLINE ZUSAMMENSETZUNGEN
COMPOSES DE CRISTAUX LIQUIDES COMPORTANT DES LIAISONS VINYLE ET COMPOSITION DE CRISTAUX LIQUIDES

(30) Priority: 20.11.1995 JP 32499395
(43) Date of publication of application: 23.12.1998
(73) Proprietor: CHISSO CORPORATION (SADAO NOGI), Osaka-shi Osaka 530 (JP)
(72) Inventor: KATO, Takashi, Ichihara-shi, Chiba 290-0003 (JP); MATSUI, Shuichi, Ichihara-shi, Chiba 290 (JP); MIYAZAWA, Kazutoshi, Yachiyo-shi, Chiba 290-0022 (JP); HACHIYA, Norihisa, Yashio-shi, Saitama 340-0815 (JP); NAKAGAWA, Etsuo, Ichihara-shi, Chiba 290 (JP)
(74) Representative: Hansen, Bernd, Dr. Dipl.-Chem.
(86) International application number: JP9603358
(87) International publication number: WO9719042

(56) References cited:
- WO-A-95/30723

## Description

The present invention relates to liquid crystalline compounds, liquid crystal compositions and liquid crystal display devices. More specifically, it relates to novel 4-ring liquid crystalline compounds having two trans-1,2-ethenylene groups having a ring therebetween in the center portion of the molecule, liquid crystal compositions comprising the compound, and liquid crystal display device comprising the liquid crystal composition.

### BACKGROUND ART

Display devices employing optical anisotropy and dielectric anisotropy of the characteristics of liquid crystalline compounds are widely used in watches, table-top calculators, word processors, and television sets, and their demand is increasing year by year. Liquid crystal phase is an intermediate one between solid and liquid phases, and is divided roughly into nematic phase, smectic phase, and cholesteric phase. Display devices employing the nematic phase are utilized most widely at present. Display mode applied to liquid crystal displays includes TN (twist nematic) mode, DS (dynamic scattering) mode, guest-host mode, and DAP mode as one having electro-optical effects. In particular, conversion of monochrome liquid crystal displays to colored ones is further progressing recently, a thin film transistor (TFT) mode and super twisted nematic (STN) mode has become a main stream in the TN-type display mode, and these display devices are being produced in a large quantity. Although a number of liquid crystalline compounds including those under research stage are known, there is not any substance which can be filled and used in display devices as sole liquid crystal substance. This is because the liquid crystalline compounds expected as materials for display devices are preferably those which exhibit a liquid crystal phase over as wide a temperature range as possible in nature, with room temperature being its center which temperature is most frequently used for display devices, and because the compounds should be sufficiently stable against environmental conditions used and should have physical properties sufficient for driving display devices. Hence, at present, a few to twenty or more liquid crystalline compounds or liquid non-crystalline compounds are mixed to prepare compositions having such properties, and are used in practice. These liquid crystal compositions are required to be stable against moisture, light, heat, and air usually present in the environment where they are used. In addition, they are required to be stable against electric field and electromagnetic radiation. Besides, liquid crystalline compounds to be mixed should be chemically stable in the environment where they are used. Further, the Liquid crystal compositions are necessary to have suitable values of physical properties such as optical anisotropy (An), dielectric anisotropy (Δε), viscosity (η), electric conductance and elastic constant ratio (K₃₃/K₁₁) (K₃₃, bend constant; K₁₁, spray elastic constant) depending on the display mode and the shape of the devices used. Also, it is important that each component in the liquid crystal compositions has good mutual solubility.

Among these physical property values, those required for liquid crystalline compounds used in the STN-type display mode particularly include low viscosity, high optical anisotropy, large elastic constant ratio K₃₃/K₁₁, and wide temperature range for the liquid crystal phase, and furthermore, low threshold voltage is also required recently to cope with low-voltage driving of display devices.

Low viscosity is an essential properties to attain high response speed. This high response speed can also be attained by thinning cell thickness. However, if cell thickness is merely thinned, display contrast is lowered and viewing angle is narrowed. This is because display contrast and viewing angle are greatly influenced by the product (Δn·d) of optical anisotropy (Δn) and cell thickness (d). For thinning cell thickness while keeping Δn·d at a desired value, liquid crystal compositions having a high Δn should be prepared and thus liquid crystalline compounds having a high Δn become necessary. Further, large elastic constant ratio K₃₃/K₁₁ makes the voltage-transmittance curve steep, thus realizing high-contrast display devices.

In general, compounds having π electrons, that is, compounds having many structures such as benzene ring, double bond, and triple bond in the molecule are known to have a high optical anisotropy. Up to now, compounds containing many triple bonds and benzene rings, for example, tolan compounds have been used to prepare liquid crystal compositions having a high optical anisotropy from 3- or 4-ring compounds. However, these compounds containing many triple bonds and benzene rings tend to be highly viscous, so they suffer from the problem that additional liquid crystalline compounds for decreasing their viscosity should be mixed with them.

As compounds having a relatively low viscosity and a high elastic constant ratio K₃₃/K₁₁, the following compounds (a) (disclosed in Japanese Patent Publication No. Hei 7-72148) and (b) (disclosed in Laid-open Japanese Patent Application No. Sho 61-215336) are known. 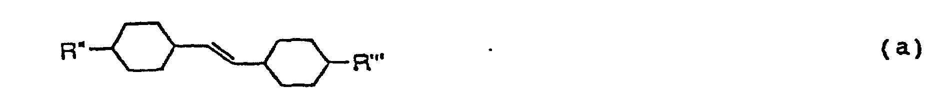 wherein R'' and R''' are different alkyl groups.

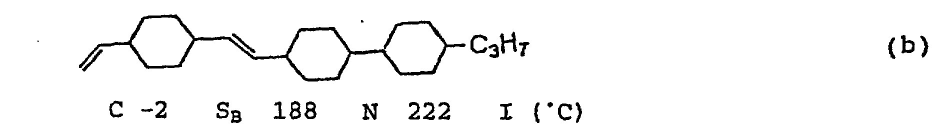

However, if compound (a) is mixed with liquid crystal compositions to increase elastic constant ratio K₃₃/K₁₁, the elastic constant ratio K₃₃/K₁₁ may increase to a certain degree, but the clearing point cannot be maintained and will be lowered. Compound (b) has a higher clearing point than compound (a), but because its viscosity is high, addition of compound (b) as a component in liquid crystal compositions would result in significant increase in viscosity. Further, as can also be seen from its transition point, smectogenecity is significantly strong, thus leading to the disadvantage that a smectic phase is developed in the compositions at a cryogenic temperature of -20 to -40 °C.

Accordingly, developments of liquid crystalline compounds having a low viscosity, high optical anisotropy, large elastic constant ratio K₃₃/K₁₁, wide temperature range of liquid crystal phase, and improved mutual solubility at low temperatures have been desired.

WO 95/30723 relates to nematic liquid crystal mixtures consisting of
(a) 20 - 90 wt.% of a specific liquid crystalline component A,
(b) 10 - 65 wt.% of a specific liquid crystalline component B,
(c) 0 - 20 wt.% of a specific liquid crystalline component D,
(d) an optically active component C,
characterized in that component B contains at least one compound of the following formula (I) 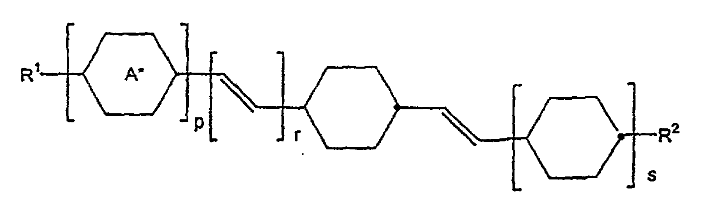 wherein R¹ represents unsubstituted or specifically substituted alkyl, alkoxy or alkenyl residues, R² represents specific alkenyl residues or straight chain alkyl, and p and r independently are 0 or 1, and s is 1 or 2. These liquid crystal compounds are used in super twisted liquid crystal displays having short response times, steep electro-optical characteristic curves and good angle dependencies.

### DISCLOSURE OF THE INVENTION

The subject of the present invention is to provide liquid crystalline compounds having a low viscosity, high optical anisotropy, large elastic constant ratio K₃₃/K₁₁, wide temperature range of liquid crystal phase, and improved mutual solubility at low temperatures; to provide liquid crystal compositions comprising the compound; and to provide liquid crystal display devices comprising the liquid crystal composition.

As a means of solving the above subject, the present invention is summarized as follows:
(1) A liquid crystalline compound expressed by the general formula (1): 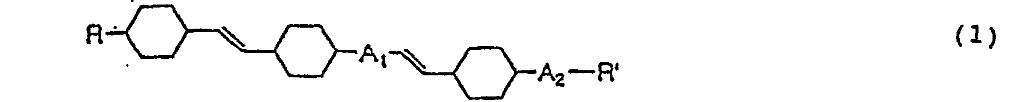 wherein R is a C₁-C₁₀ alkyl group or a C₂-C₁₀ alkenyl group; R' is a C₁-C₁₀ alkyl group, a C₂-C₁₀ alkenyl group, F, Cl, CN, OCF₃, CF₃, OCF₂H, CF₂H, or CFH₂; A₁ and A₂ are independently trans-1,4-cyclohexylene group, 1,4-phenylene group in which one or more hydrogen atoms on the 6-membered ring may be replaced by a halogen atom, or single bond, provided that A₁ and A₂ are selected such that the number of rings in the compound of formula (1) is four and each of the atoms constituting the compound may be replaced by an isotope thereof.
(2) The liquid crystalline compound recited in item (1) wherein A₁ is a trans-1,4-cyclohexylene group and A₂ is single bond in the general formula (1).
(3) The liquid crystalline compound recited in item (1) wherein A₁ is single bond and A₂ is trans-1,4-cyclohexylene group or 1,4-phenylene group in the general formula (1).
(4) A liquid crystal composition comprising at least two components and comprising at least one liquid crystalline compound described in any one of items (1) to (3).
(5) A liquid crystal composition comprising, as a first component, at least one liquid crystalline compound described in any one of items (1) to (3) and comprising, as a second component, at least one compound selected from the group of consisting of the compounds expressed by any one of the general formulae (2), (3), and (4): 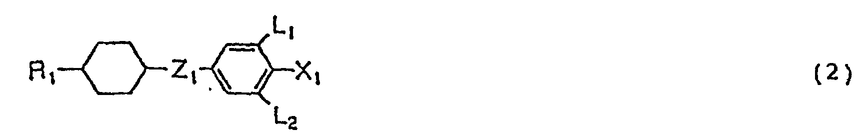 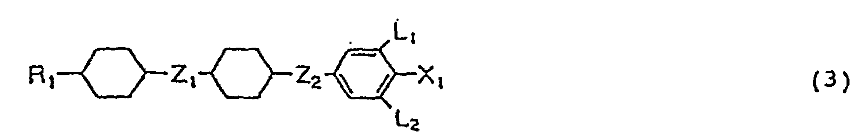 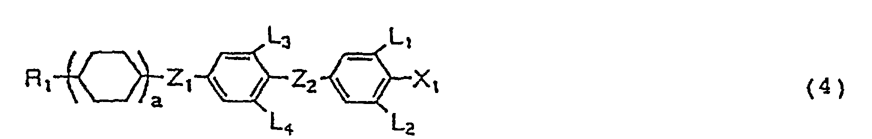 wherein R₁ is a C₁-C₁₀ alkyl group; X₁ is F, Cl, OCF₃, OCF₂H, CF₃, CF₂H, or CFH₂; L₁, L₂, L₃, and L₄ are independently H or F; Z₁ and Z₂ are independently -(CH₂)₂-, -CH=CH-, or single bond; and a is 1 or 2.
(6) A liquid crystal composition comprising, as a first component, at least one liquid crystalline compound described in any one of items (1) to (3) and comprising, as a second component, at least one compound selected from the group consisting of the compounds expressed by any one of the general formulae (5), (6), (7), (8), and (9) : 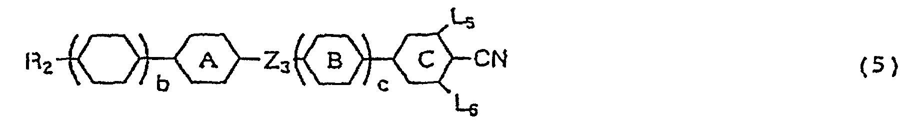 wherein R₂ is F, a C₁-C₁₀ alkyl group or a C₂-C₁₀ alkenyl group, one or more non adjacent methylene (-CH₂-) groups in the alkyl group or alkenyl group may be replaced by oxygen atom (-O-); ring A is trans-1,4-cylcohexylene group, 1,4-phenylene group, pyrimidine-2,5-diyl group, or 1,3-dioxane-2,5-diyl group; ring B is trans-1,4-cyclohexylene group, 1,4-phenylene group, or pyrimidine-2,5-diyl group; ring C is trans-1,4-cyclohexylene group or 1,4-phenylene group; Z₃ is -(CH₂)₂-, -COO- or single bond; L₅ and L₆ are independently H or F; and b and c are independently 0 or 1; 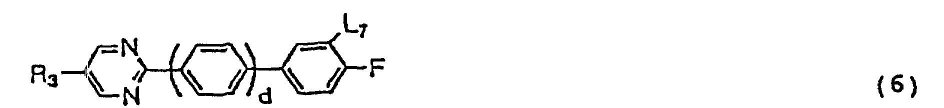 wherein R₃ is a C₁-C₁₀ alkyl group; L₇ is H or F; and d is 0 or 1; 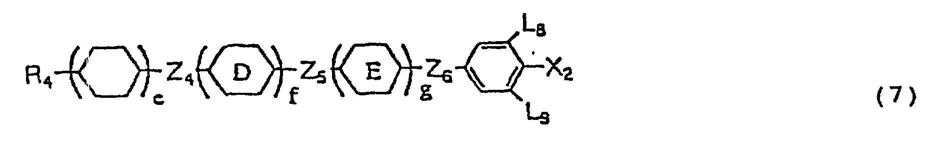 wherein R₄ is a C₁-C₁₀ alkyl group; rings D and E are independently trans-1,4-cyclohexylene group or 1,4-phenylene group; Z₄ and Z₅ are independently -COO- or single bond; Z₆ is -COO- or -C≡C-; L₈ and L₉ are independently H or F; X₂ is F, OCF₃, OCF₂H, CF₃, CF₂H, or CFH₂; and e, f and g are independently 0 or 1; 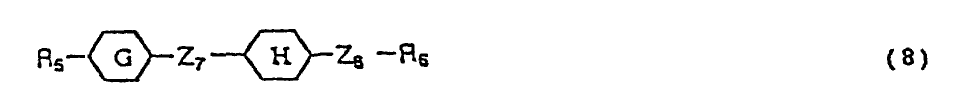 wherein R₅ and R₆ are independently a C₁-C₁₀ alkyl group or a C₂-C₁₀ alkenyl group, one or more non adjacent methylene (-CH₂-) groups in the alkyl group or alkenyl group may be replaced by oxygen atom (-O-); ring G is trans-1,4-cyclohexylene group, 1,4-phenylene group, or pyrimidine-2,5-diyl group; ring H is trans-1,4-cyclohexylene group or 1,4-phenylene group; Z₇ is -C≡C-, -COO-, - (CH₂)₂-, -CH=CH-C≡C-, or single bond; and Z₈ is -COO- or single bond; and 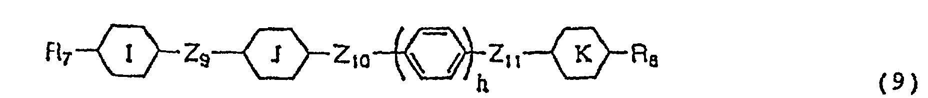 wherein R₇ and R₈ are independently a C₁-C₁₀ alkyl group or a C₂-C₁₀ alkenyl group, one or more non adjacent methylene (-CH₂-) groups in the alkyl group or alkenyl group may be replaced by oxygen atom (-O-); ring I is trans-1,4-cyclohexylene group, 1,4-phenylene group, or pyrimidine-2,5-diyl group; ring J is trans-1,4-cyclohexylene group, 1,4-phenylene group in which one or more hydrogen atoms on the ring may be replaced by F, or pyrimidine-2,5-diyl group; ring K is trans-1,4-cyclohexylene group or 1,4-phenylene group; Z₉ and Z₁₁ are independently -COO-, -(CH₂)₂-, or single bond; Z₁₀ is -CH=CH-, -C≡C-, -COO- or single bond; and h is 0 or 1.
(7) A liquid crystal composition comprising, as a first component, at least one liquid crystalline compound described in any one of items (1) to (3), comprising, as a part of a second component, at least one compound selected from the group consisting of the compounds expressed by any one of the general formulae (2), (3) and (4), and comprising, as another part of the second component, at least one compound selected from the group consisting of the compounds expressed by any one of the general formulae (5), (6), (7), (8), and (9).
(8) A liquid crystal display device including a liquid crystal composition comprising at least two components and comprising at least one liquid crystalline compound described in any one of items (1) to (3).
(9) A liquid crystal display device including the liquid crystal composition described in item (5).
(10) A liquid crystal display device including the liquid crystal composition described in item (6).
(11) A liquid crystal display device including the liquid crystal composition described in item (7).

### BRIEF DESCRIPTION OF THE DRAWING

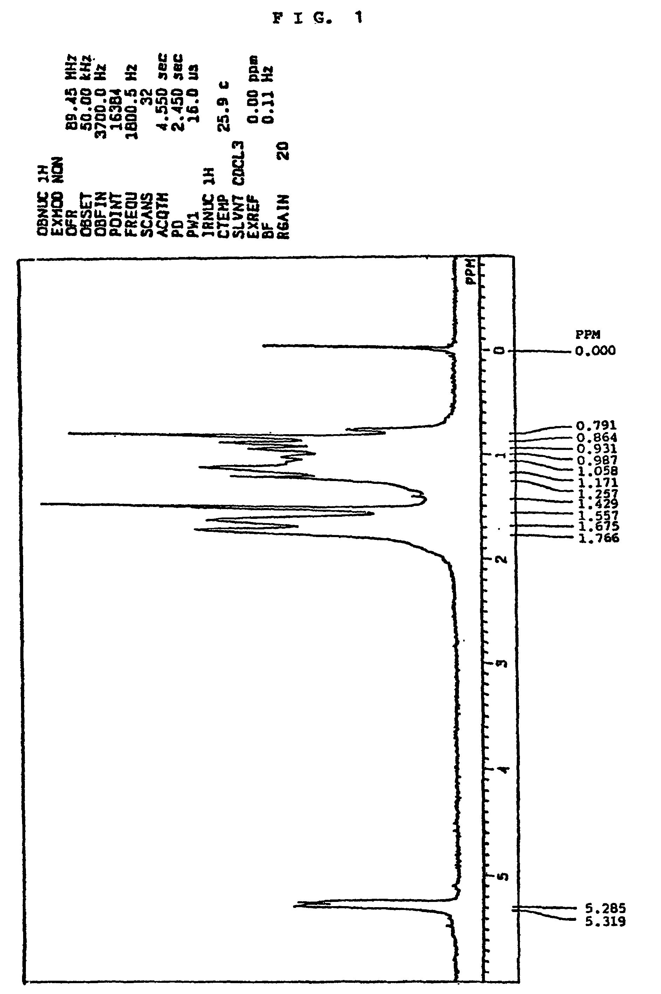

Fig. 1 shows a nuclear magnetic resonance spectrum of trans-1,4-bis-((E)-2-(tans-4-propylcyclohexyl)ethenyl)cyclohexane prepared in Example 1.

### BEST MODE FOR CARRYING OUT THE INVENTION

The liquid crystalline compounds of the present invention are 4-ring derivatives having two trans-1,2-ethenylene groups having a ring therebetween in the center portion of the molecule as shown in the general formula (1).

These liquid crystalline compounds are characterized in that they are not only very stable physically and chemically under the conditions where display devices are used, but also are excellent in mutual solubility in liquid crystal compositions even at low temperatures, exhibit a liquid crystal phase over a wide temperature range, have a low viscosity, a high optical anisotropy and a large elastic constant ratio K₃₃/K₁₁. Further, desired physical properties can be arbitrarily controlled by suitably selecting the ring structures, bonding groups or side chain structure of the molecular elements. Therefore, when the compound of the present invention is used as component of liquid crystal compositions, it is possible to provide novel liquid crystal compositions which a) exhibit such characteristics that:
1) high contrast can be obtained because optical anisotropy and elastic constant ratio K₃₃/K₁₁ are improved;
2) viscosity is lowered while the clearing point of the resulting liquid crystal composition is maintained or raised; and
3) stable nematic liquid crystal compositions which do not separate crystals or develop smectic phase even at cryogenic temperatures can be prepared,
   b) are stable against the external environment, and c) have a high response speed and high-contrast; and to provide liquid crystal display devices.

The compounds of the present invention expressed by the general formula (1) are classified into the following general formulae (1a) to (1d):

R-Cyc-V-Cyc-Cyc-V-Cyc-R' (1a)

R-Cyc-V-Cyc-Phe-V-Cyc-R' (1b)

R-Cyc-V-Cyc-V-Cyc-Cyc-R' (1c)

R-Cyc-V-Cyc-V-Cyc-Phe-R' (1d)

wherein Cyc is trans-1,4-cyclohexylene group; Phe is 1,4-phenylene group in which one or more hydrogen atoms on the 6-membered ring may be replaced by a halogen atom; V is trans-1,2-ethenylene group; R is a C₁-C₁₀ alkyl group or a C₂-C₁₀ alkenyl group; R' is a C₁-C₁₀ alkyl group, a C₂-C₁₀ alkenyl group, F, Cl, CN, OCF₃, CF₃, OCF₂, CF₂H, or CFH₂.

Particularly preferable compounds among the compounds expressed by one of the general formulae (1a) to (1d) include compounds expressed by the following general formulae (1-1) to (1-3): 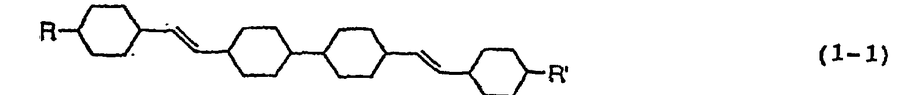 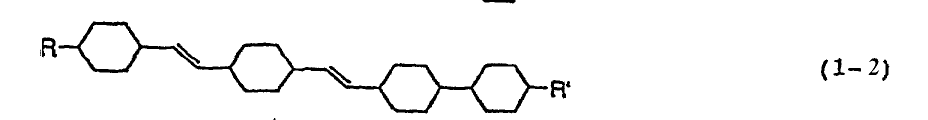 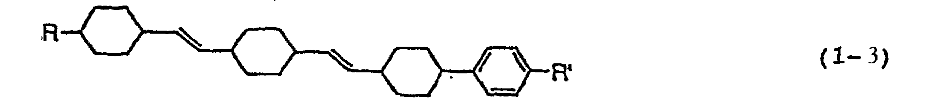 wherein R is a C₁-C₁₀ alkyl group or a C₂-C₁₀ alkenyl group; R' is a C₁-C₁₀ alkyl group, a C₂-C₁₀ alkenyl group, F, Cl, CN, OCF₃, CF₃, OCF₂H, CF₂H, or CFH₂.

Specifically, R is preferably methyl, ethyl, 1-propyl, 1-butyl, 1-pentyl, 1-hexyl, 1-heptyl, ethenyl, 1-propenyl, 2-propenyl, 1-butenyl, 2-butenyl, 3-butenyl, 1-pentenyl, 2-pentenyl, 3-pentenyl and 4-pentenyl, and R' is preferably methyl, ethyl, 1-propyl, 1-butyl, 1-pentyl, 1-hexyl, 1-heptyl, ethenyl, 1-propenyl, 2-propenyl, 1-butenyl, 2-butenyl, 3-butenyl, 1-pentenyl, 2-pentenyl, 3-pentenyl, 4-pentenyl, F, Cl, CN, OCF₃, OCF₂H, and CF₃. Among these, the compounds where R and R' are 1-propyl, 1-pentyl, or 1-heptyl are particularly preferable.

### Process for Preparing the Compounds:

The compounds of the present invention expressed by the general formula (1) can readily be synthesized by suitable selection and the use in combination of ordinary chemical procedures of organic synthesis (for example, procedures described in Organic Synthesis, Organic Reactions, and Jikken Kagaku Kouza (Course of Chemical Experiment)).

Hereinafter, an example of the processes for synthesizing the compounds expressed by the general formula (1) is described.

According to known methods using the Wittig reaction (e.g. a method described in Organic Reactions, vol. 14, ch. 3), ketone derivative (1) is reacted with methoxymethyltriphenyl phosphonium halide in the presence of a base such as sodium methylate, potassium-t-butoxide (t-BuOK), and butyl-lithium in an ether type solvent such as tetrahydrofuran (THF) and diethyl ether, and then reacted with hydrochloric acid in acetone to obtain Compound (2).

A mineral acid such as hydrochloric acid and sulfuric acid, or an organic acid such as formic acid, acetic acid, and p-toluenesulfonic acid is reacted with Compound (2) to convert it into an aldehyde derivative, and then pyridinium dichromate (PDC), pyridinium chlorochromate (PCC), or the like is reacted with the aldehyde derivative in methylene chloride to produce a carboxylic acid derivative, and this carboxylic acid derivative is converted into an ester derivative (3) by reacting with thionyl chloride in a lower saturated alcohol.

By the Wittig reaction in the same manner as described above, this ester derivative (3) is reacted with methoxymethyl-triphenyl phosphonium halide and then reacted with hydrochloric acid in acetone to obtain Compound (4).

The Wittig reaction of Compound (4) with Wittig reagent (5) gives Compound (6).

Compound (6) is reacted with lithium aluminum hydride, diisobutylaluminum hydride, or the like in an ether type solvent such as THF and diethyl ether to convert the compound into an alcohol derivative and then this alcohol derivative is oxidized with PCC to convert into a ketone derivative, and this ketone derivative is subjected to Wittig reaction with Wittig reagent (7) to obtain Compound (8).

Compound (9) is obtained by reacting compound (8) with 3-chloroperbenzoic acid (mCPBA) in the presence of a base such as potassium carbonate, sodium carbonate etc. in methylene chloride.

Compound (9) is brominated by reaction with triphenylphosphine dibromide in toluene to obtain Compound (10). Further, Compound (10) is reacted with zinc in acetic acid to produce Compound (1) of the present invention.

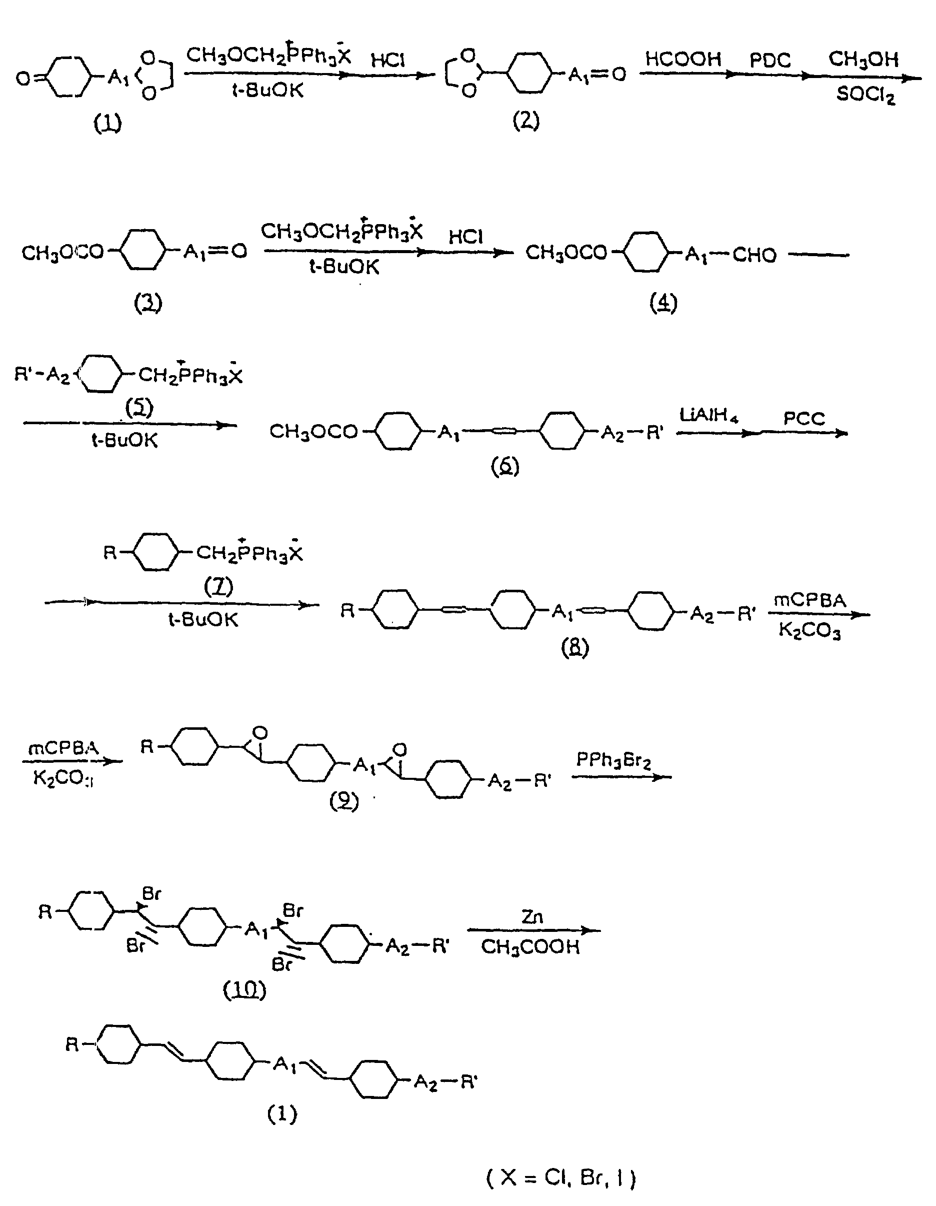

According to the method described above, the following compounds can be produced.

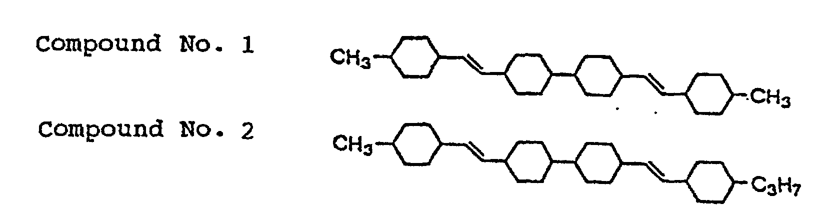

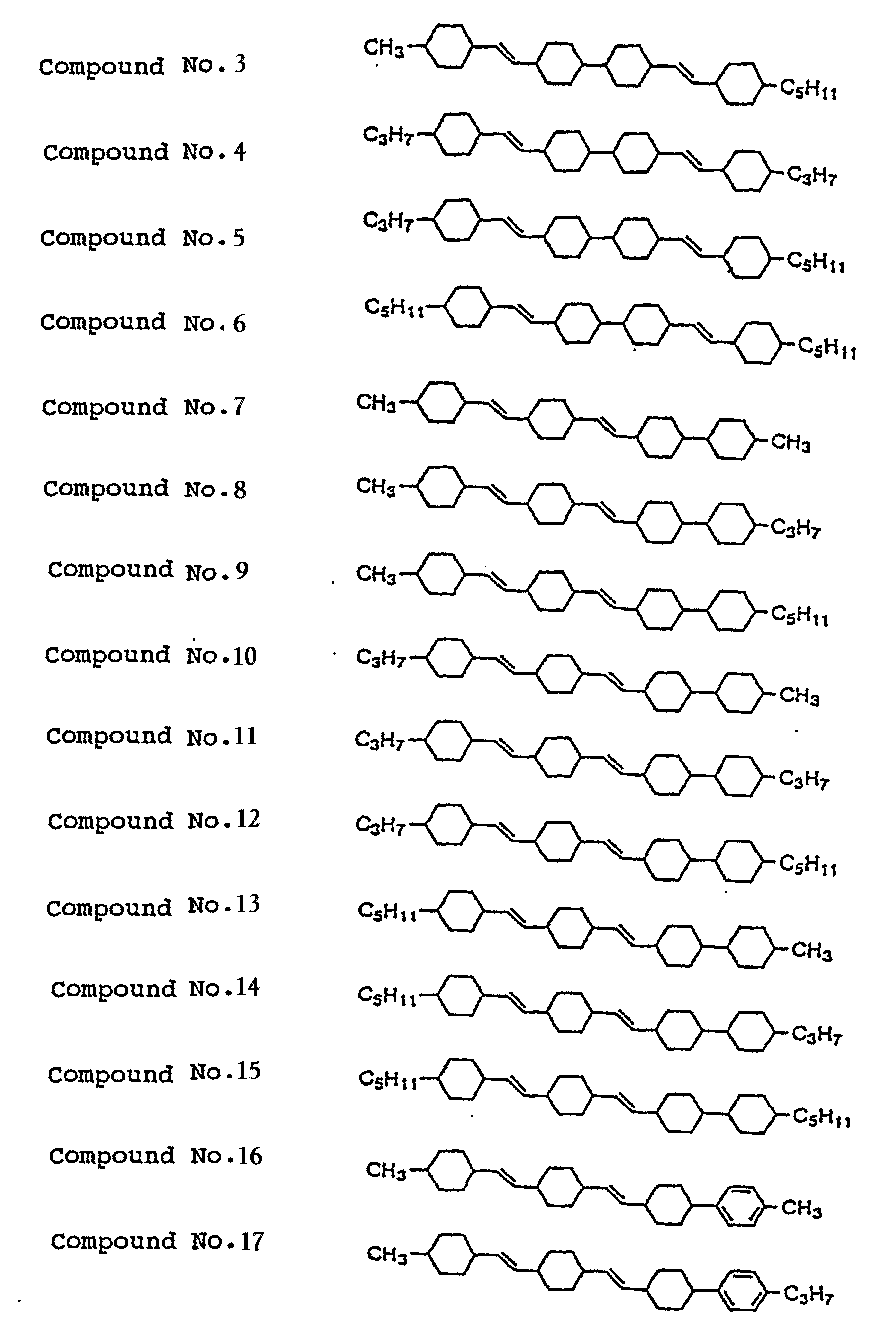

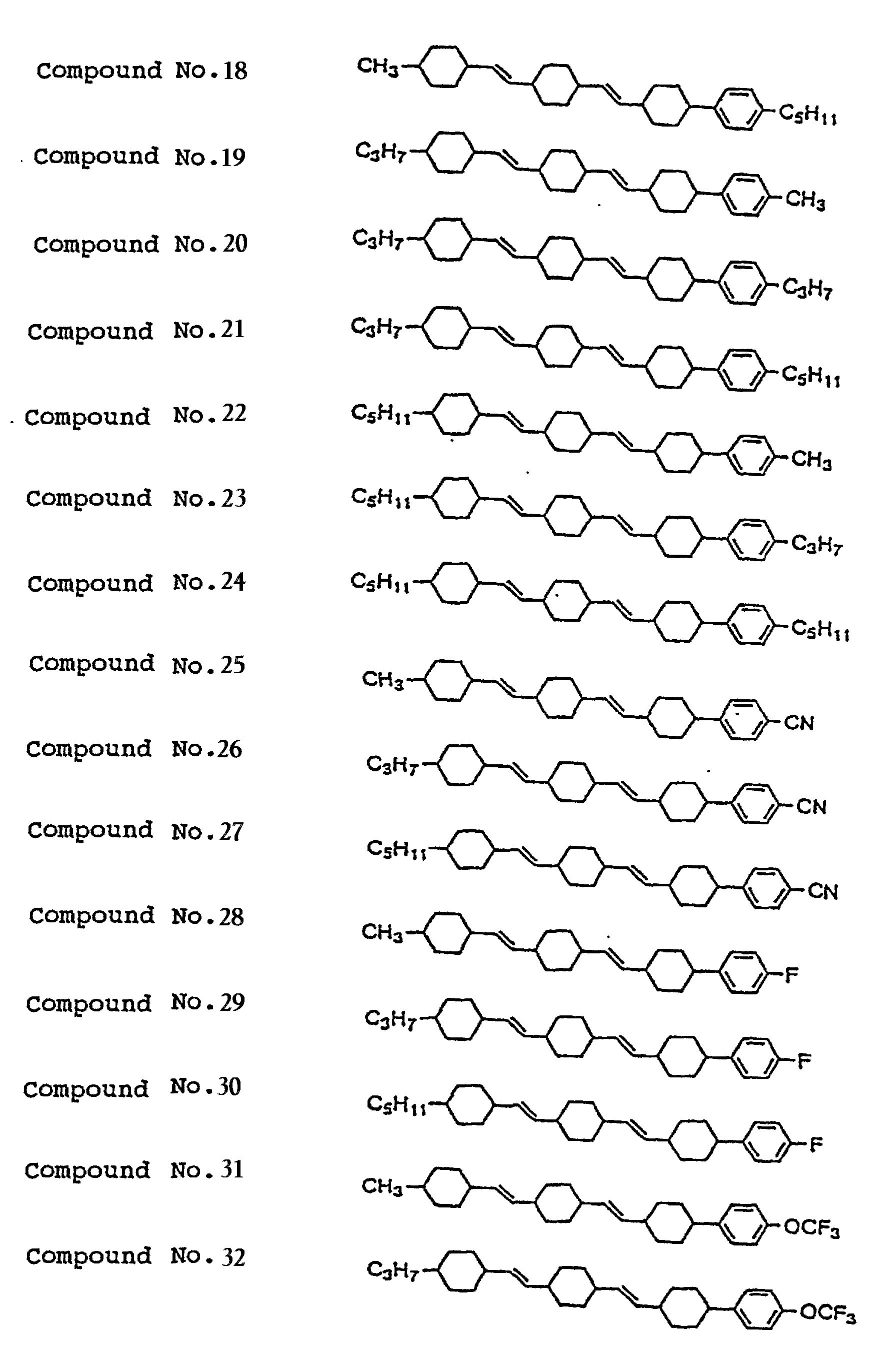

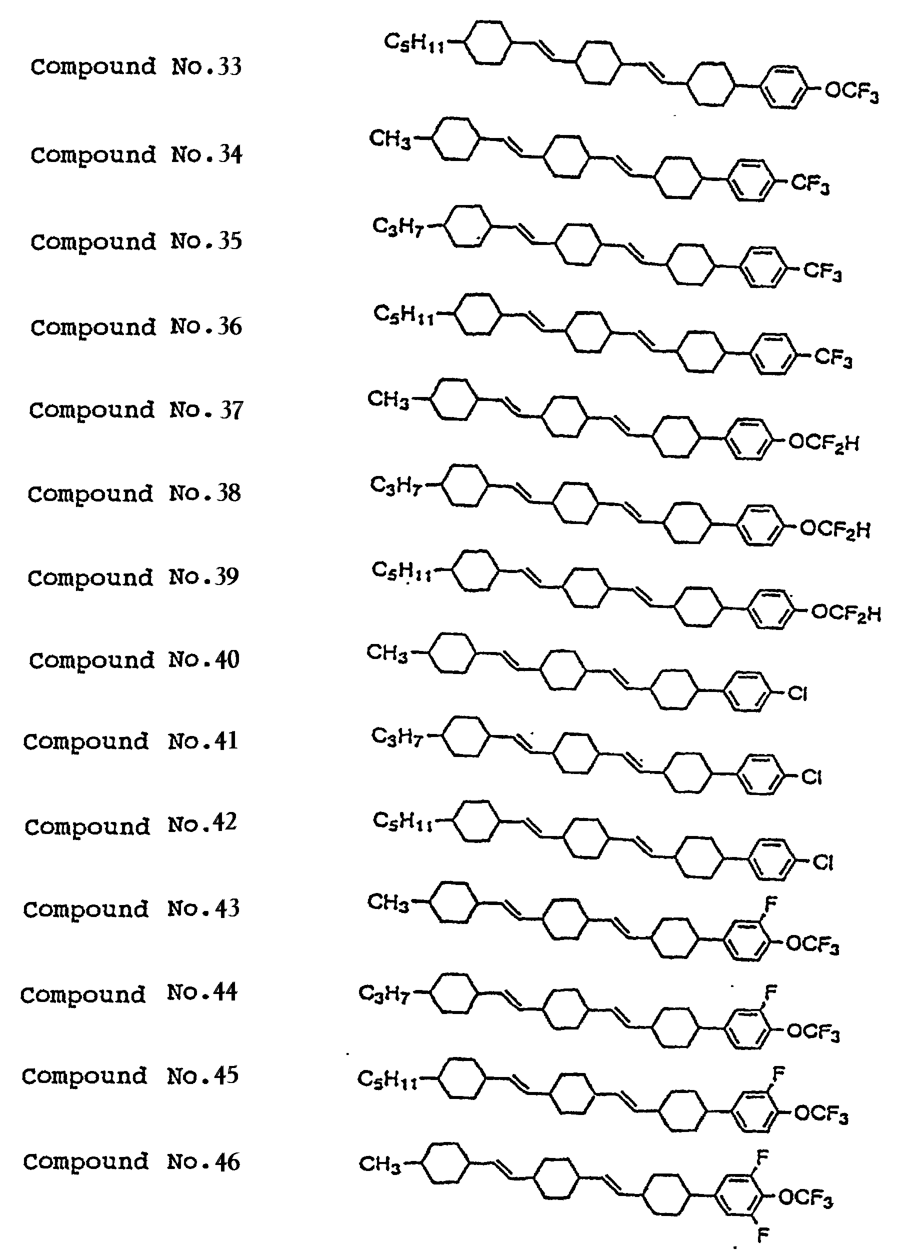

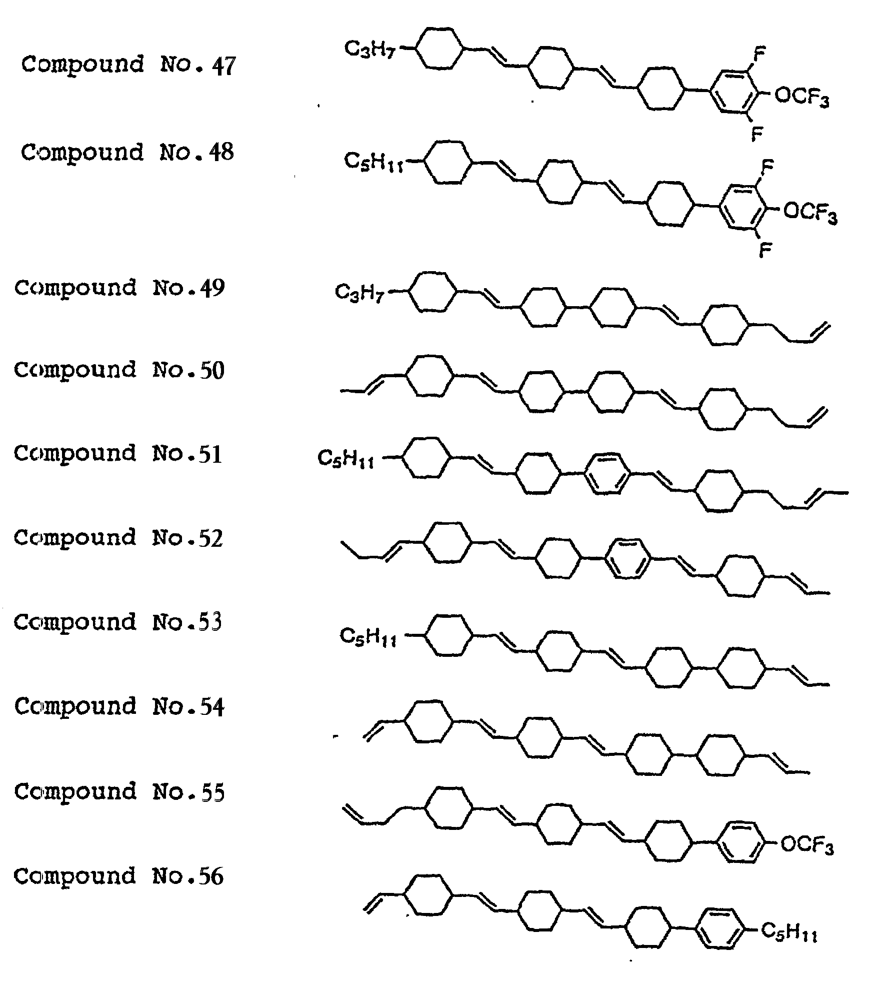

Any of the resulting liquid crystalline compounds of the present invention expressed by the general formula (1) exhibit nematic phase over a wide temperature range, have a low viscosity, high optical anisotropy, and large elastic constant ratio K₃₃/K₁₁, are readily mixed with various other liquid crystal materials, and are remarkably excellent as component of nematic liquid crystal compositions suitable for STN type display mode.

The liquid crystal compositions according to the present invention comprise at least one compound expressed by the general formula (1) preferably in a ratio of 0.1 to 99.9% by weight in order to develop superior characteristics.

Other compounds used in combination with the compounds expressed by the general formula (1) include a group of compounds expressed by one of the general formulae (2) to (9).

Although the liquid crystal compositions provided according to the present invention may comprise only a first component comprising at least one liquid crystalline compound expressed by the general formula (1), the compositions of the present invention are preferably a mixture of the first component with at least one compound (hereinafter referred to as second component A) selected from the group consisting of the compounds expressed by any one of the general formula (2), (3) and (4) and/or at least one compound (hereinafter referred to as second component B) selected from the group consisting of the compounds expressed by any one of the general formula (5), (6), (7), (8) and (9), as a second component.

For the purpose of adjusting threshold voltage, temperature range of liquid crystal phase, optical anisotropy, dielectric anisotropy, and viscosity, a known compound can be mixed as a third component with these liquid crystal compositions.

Preferable examples of compounds expressed by the general formulae (2), (3) or (4) include compounds expressed by one of the general formulae (2-1) to (2-15), general formulae (3-1) to (3-48), and general formulae (4-1) to (4-55) as follows: 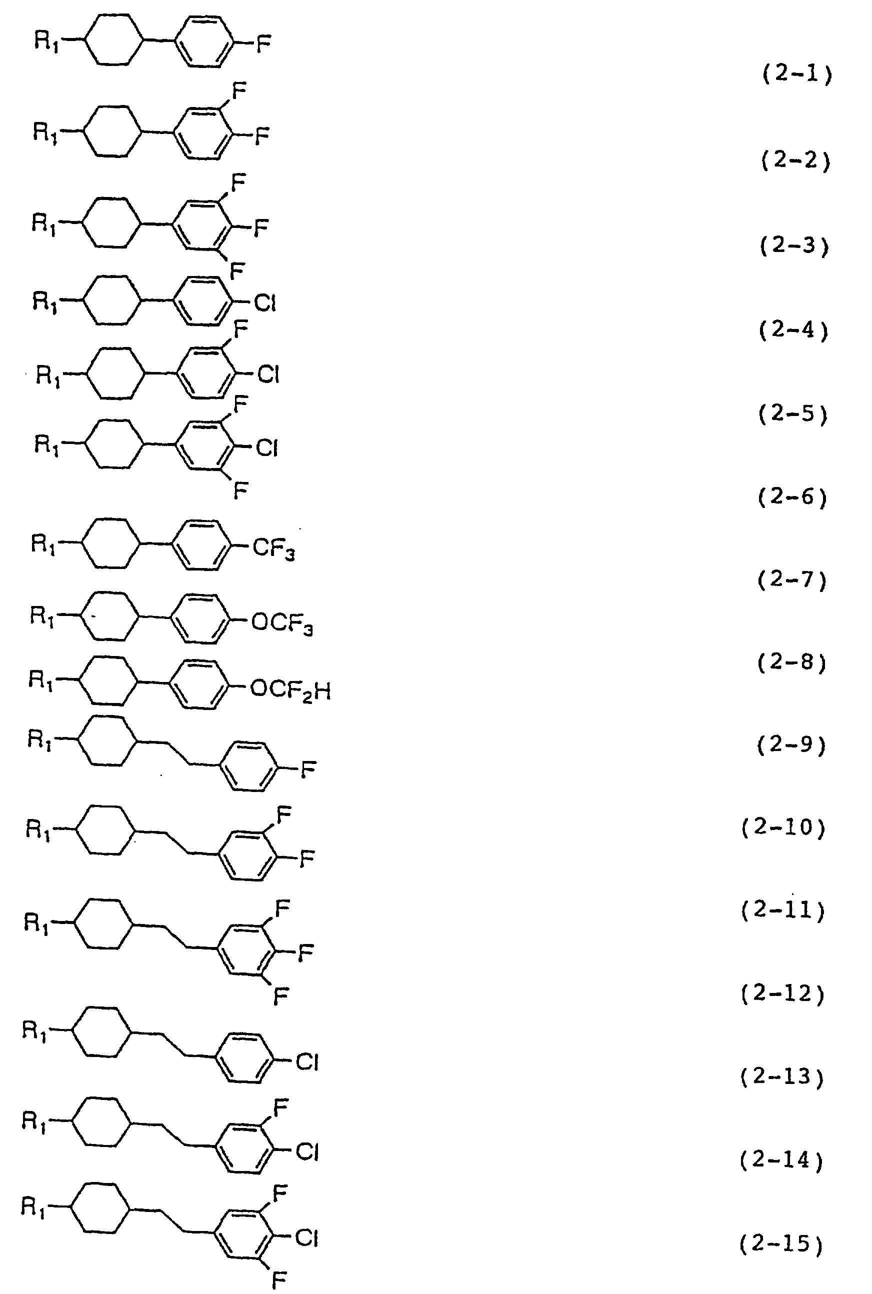 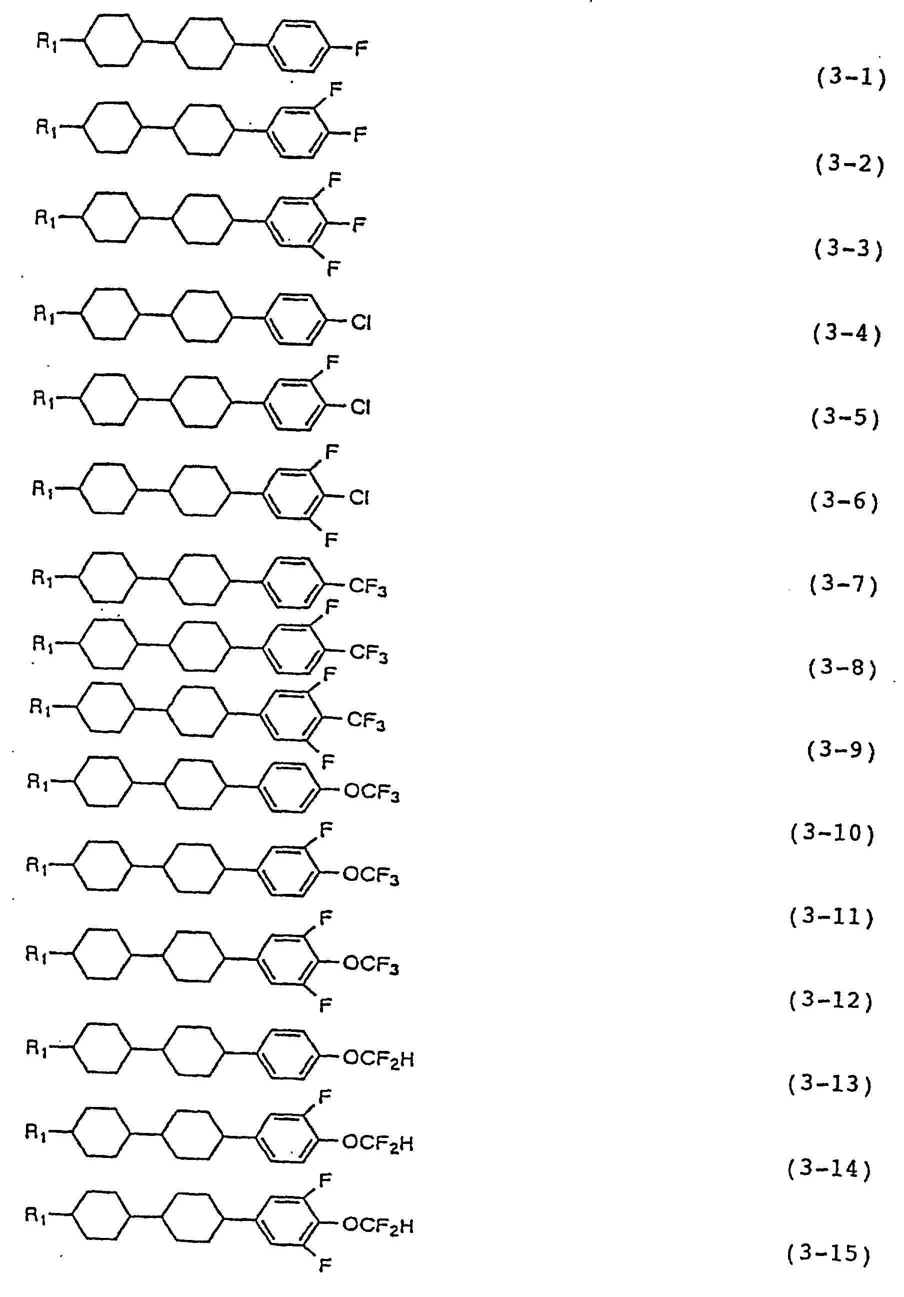 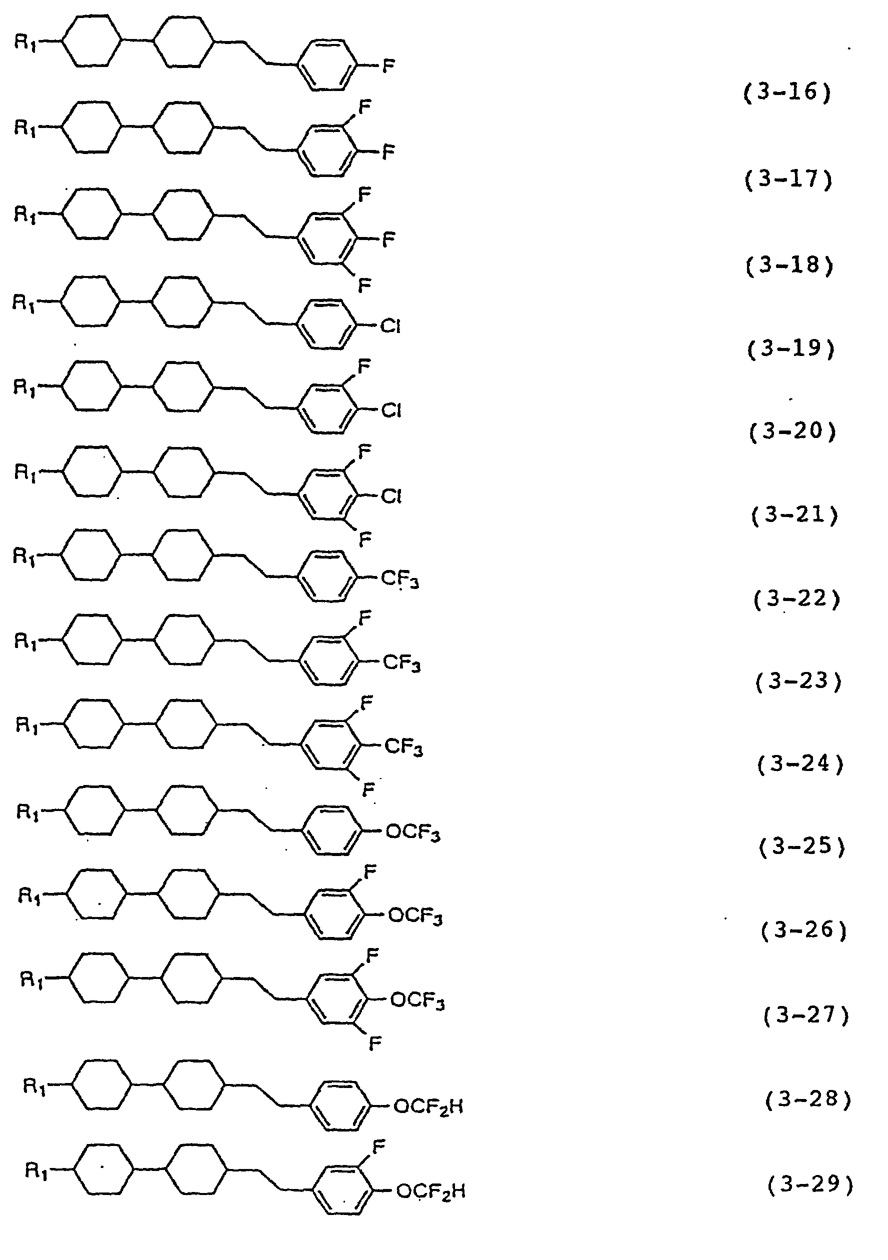 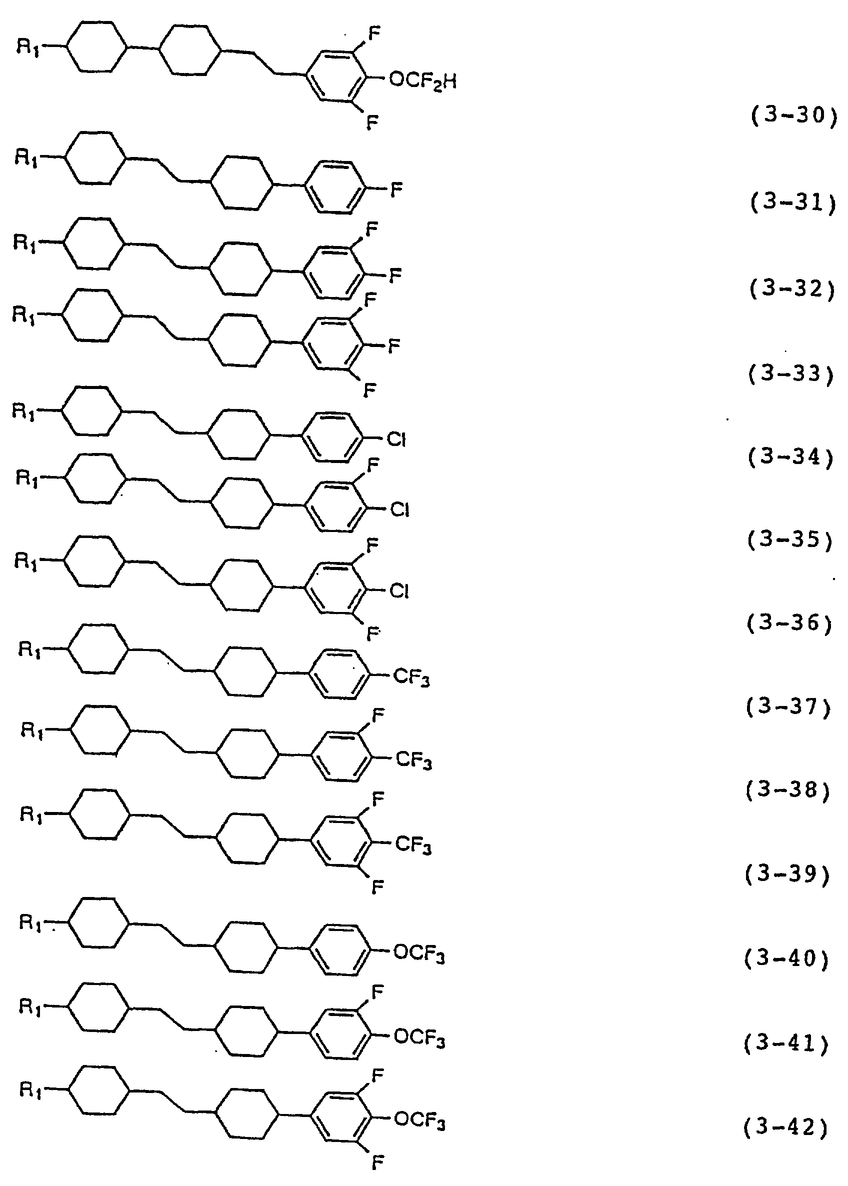 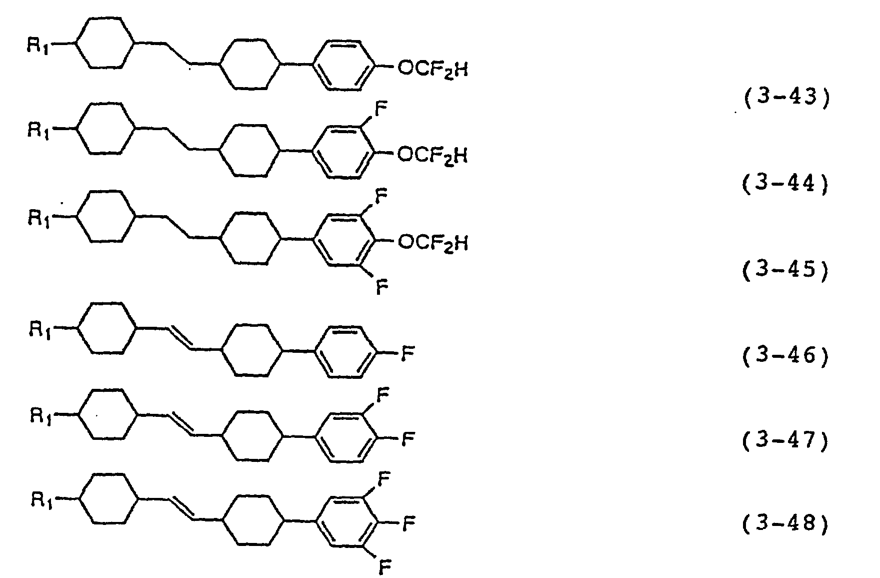 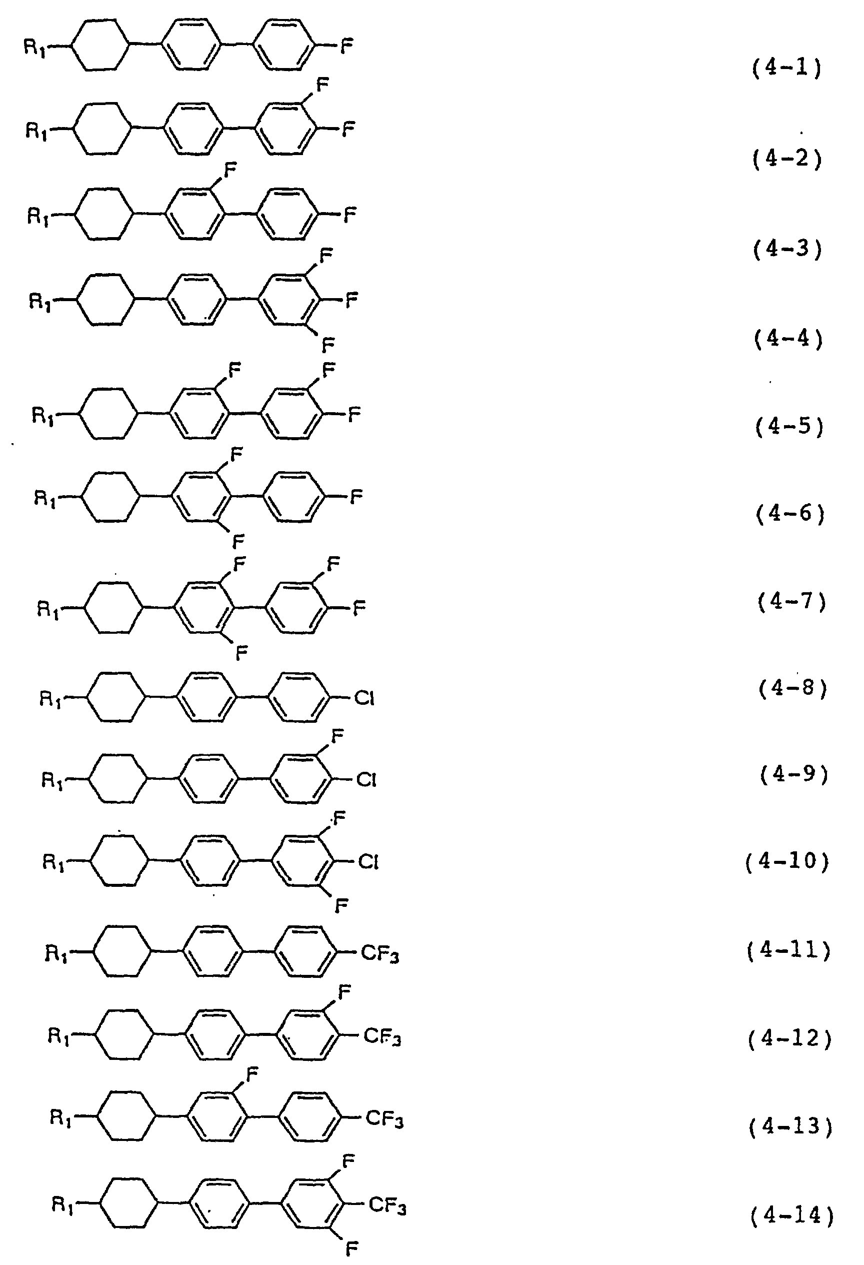 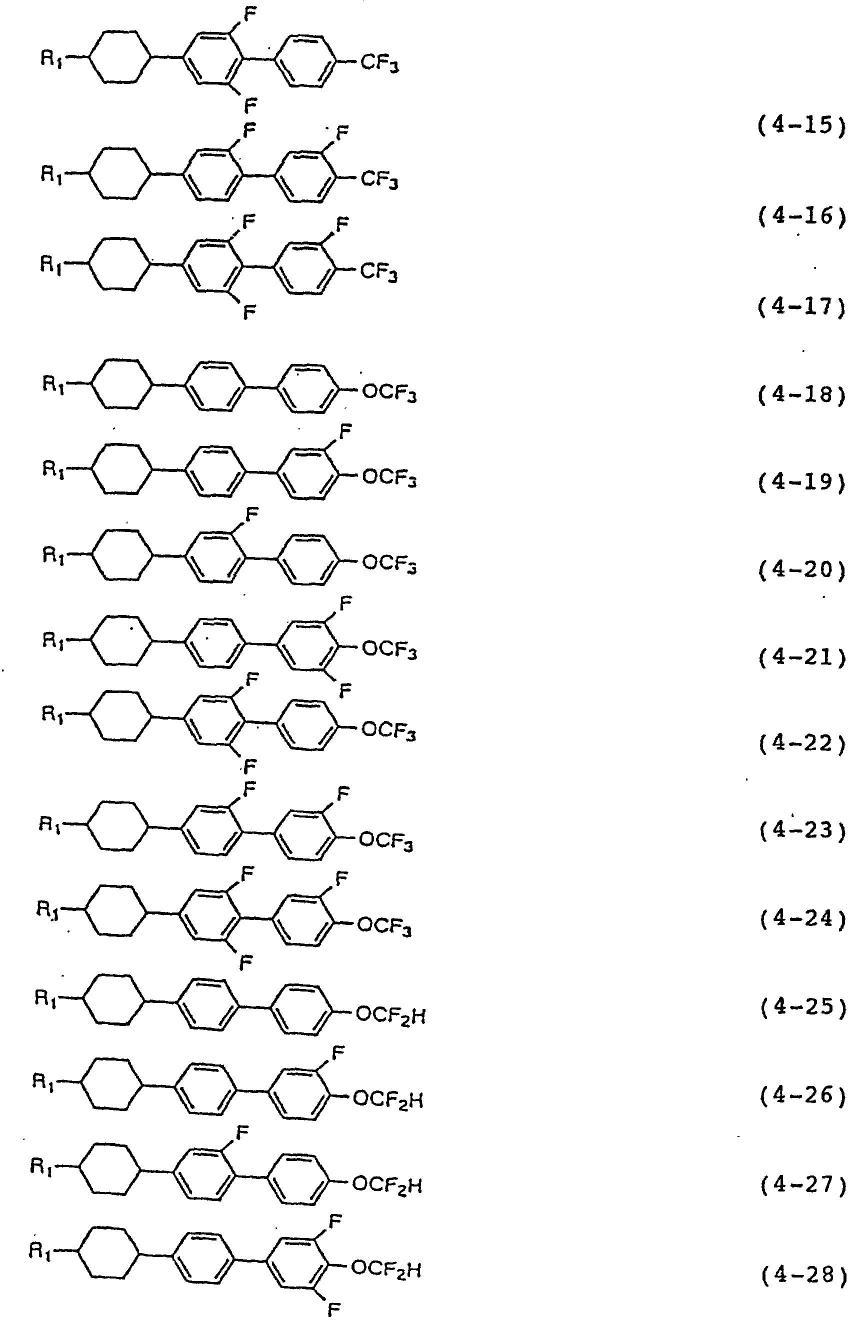 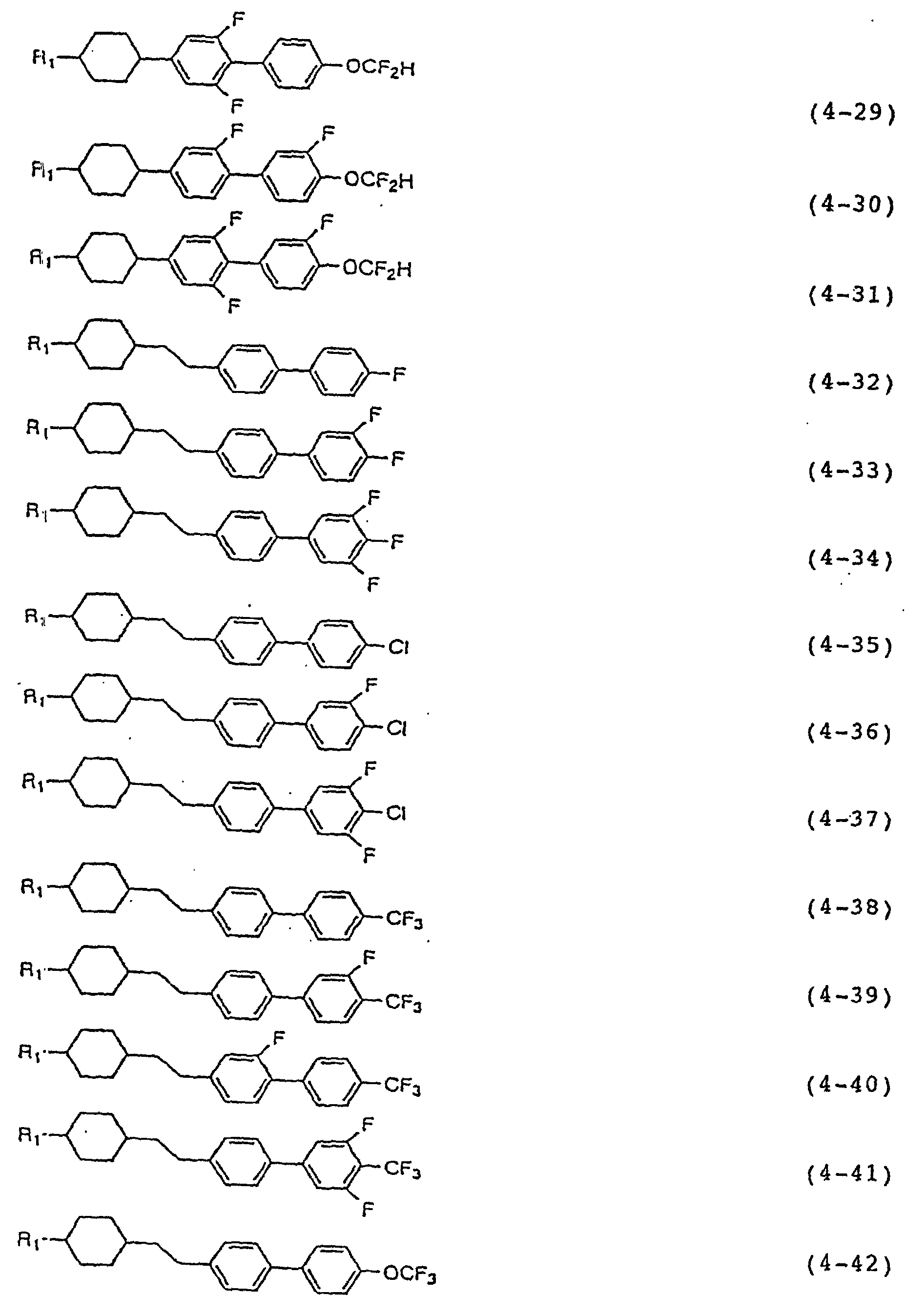 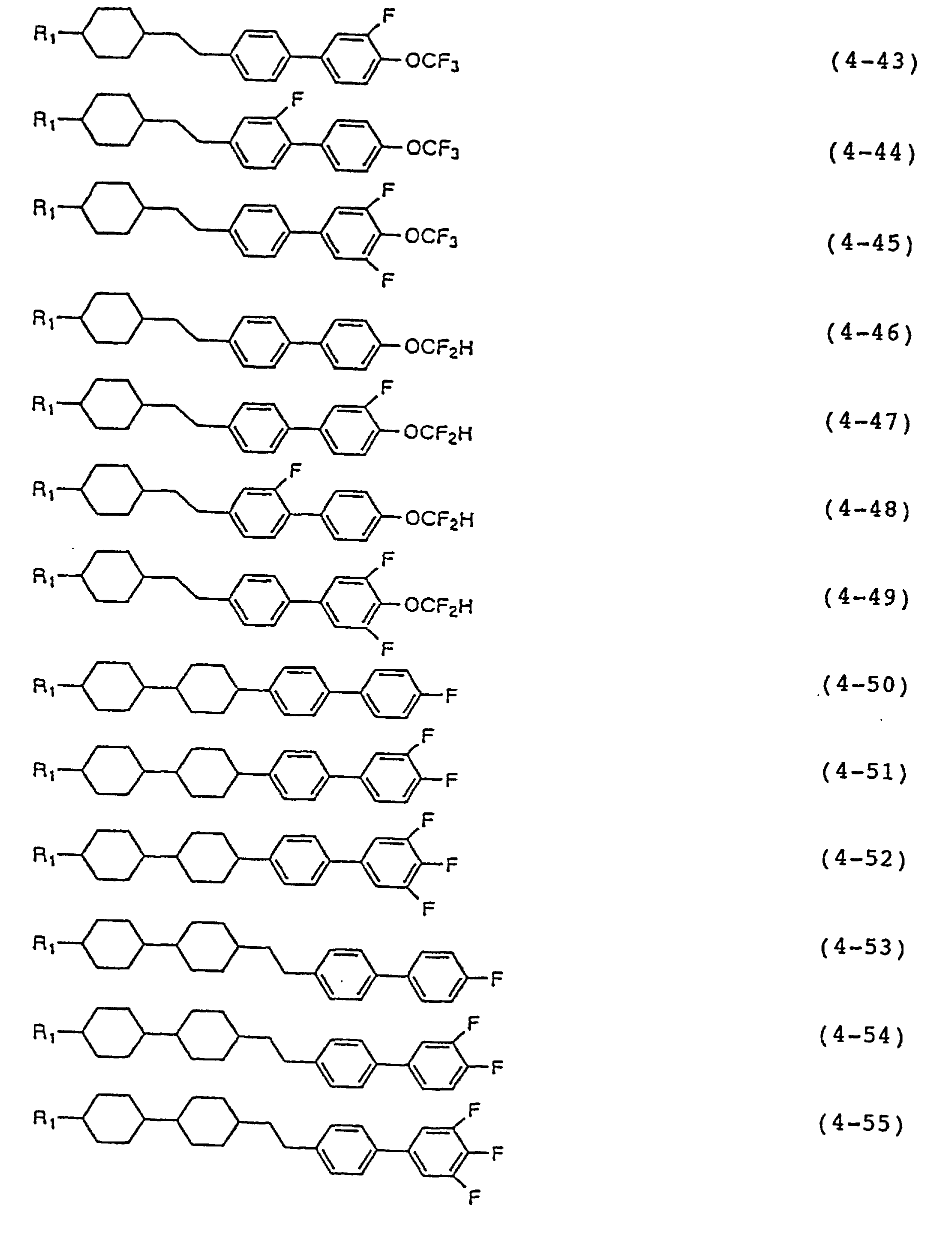 (wherein R₁ has the same meaning as in the general formula (2) shown above.)

The compounds expressed by one of the general formulae (2) to (4) have a positive dielectric anisotropy and are considerably superior in thermal stability and chemical stability. The amount of these compounds to be used in the liquid crystal composition of the present invention is suitably in the range of 1 to 99 % by weight, preferably 10 to 97 % by weight, and more desirably 40 to 95 % by weight, based on the total amount of the liquid crystal composition.

Preferred examples of compounds expressed by the general formula (5), (6) or (7) include compounds expressed by one of the general formulae (5-1) to (5-24), general formulae (6-1) to (6-3) and general formulae (7-1) to (7-28) as follows: 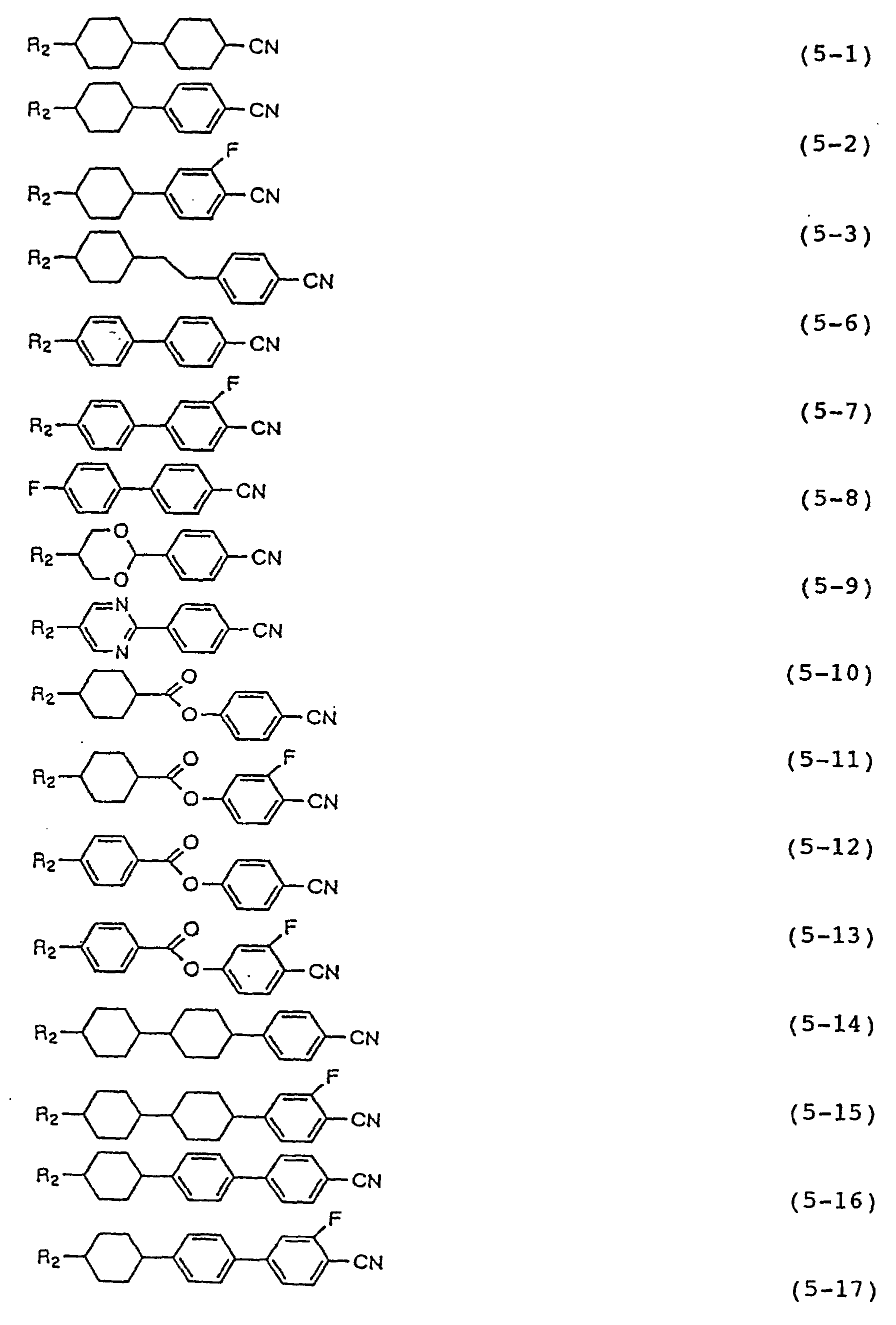 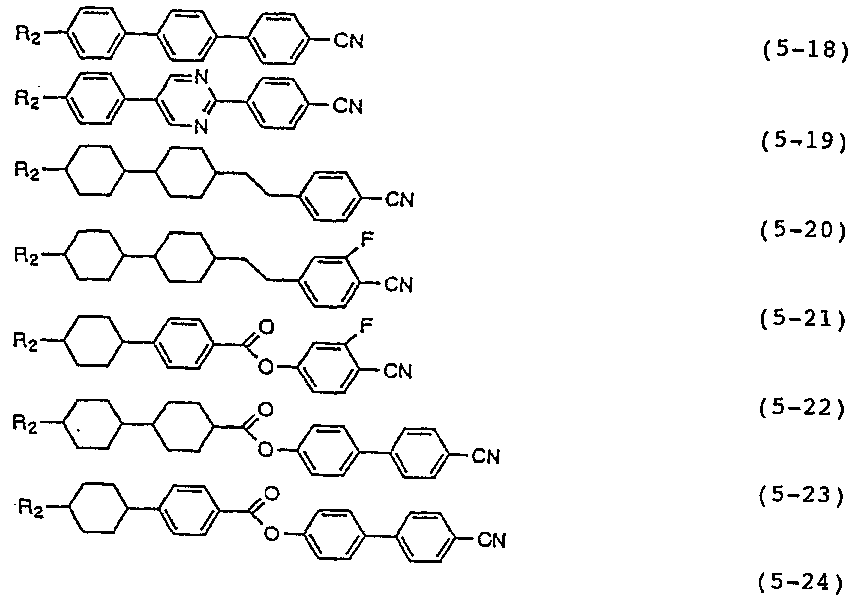 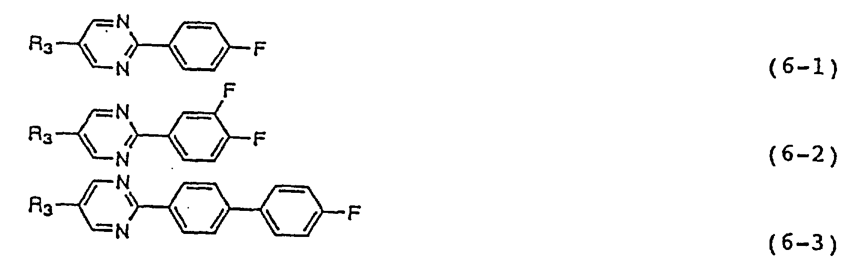 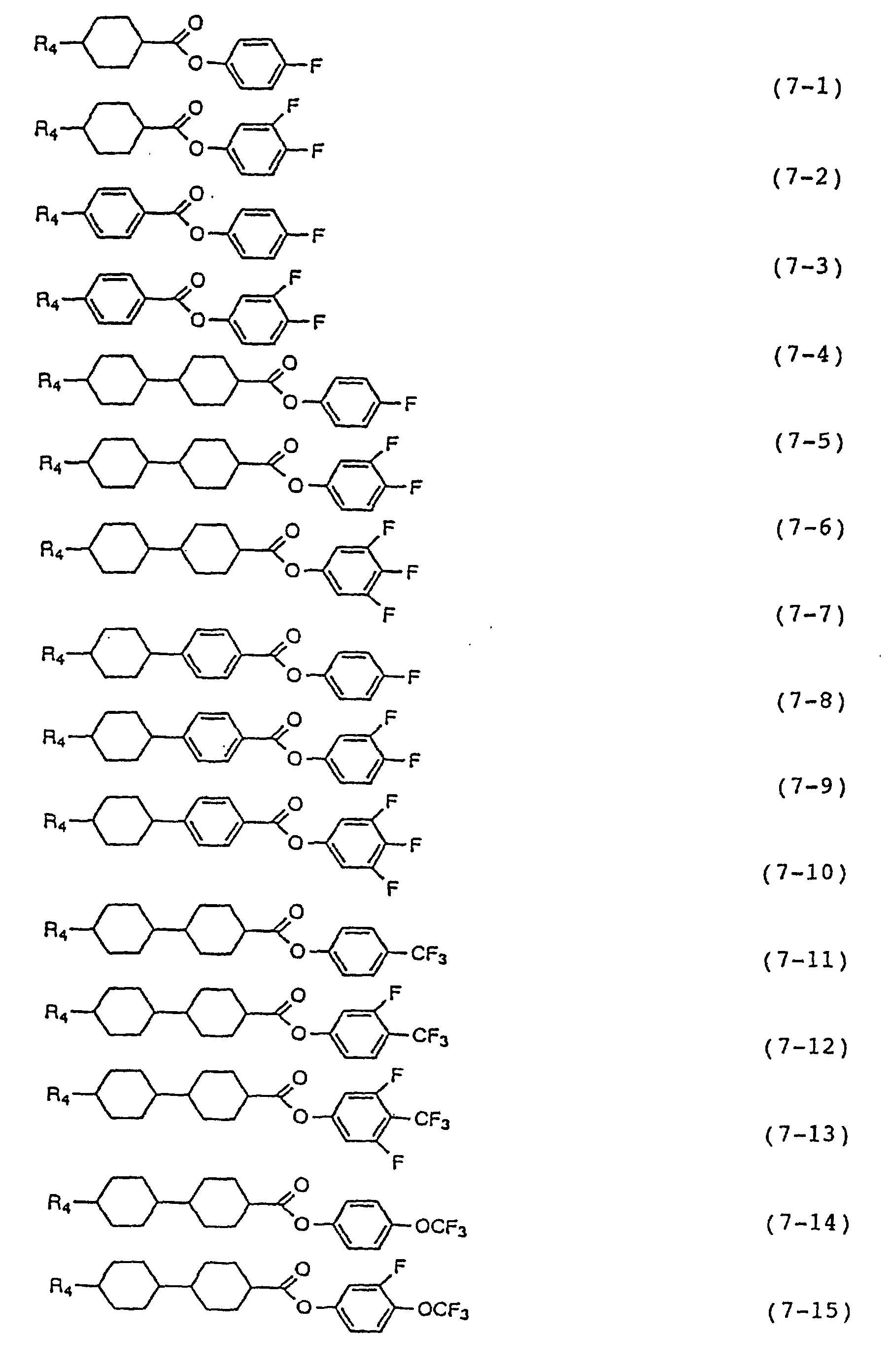 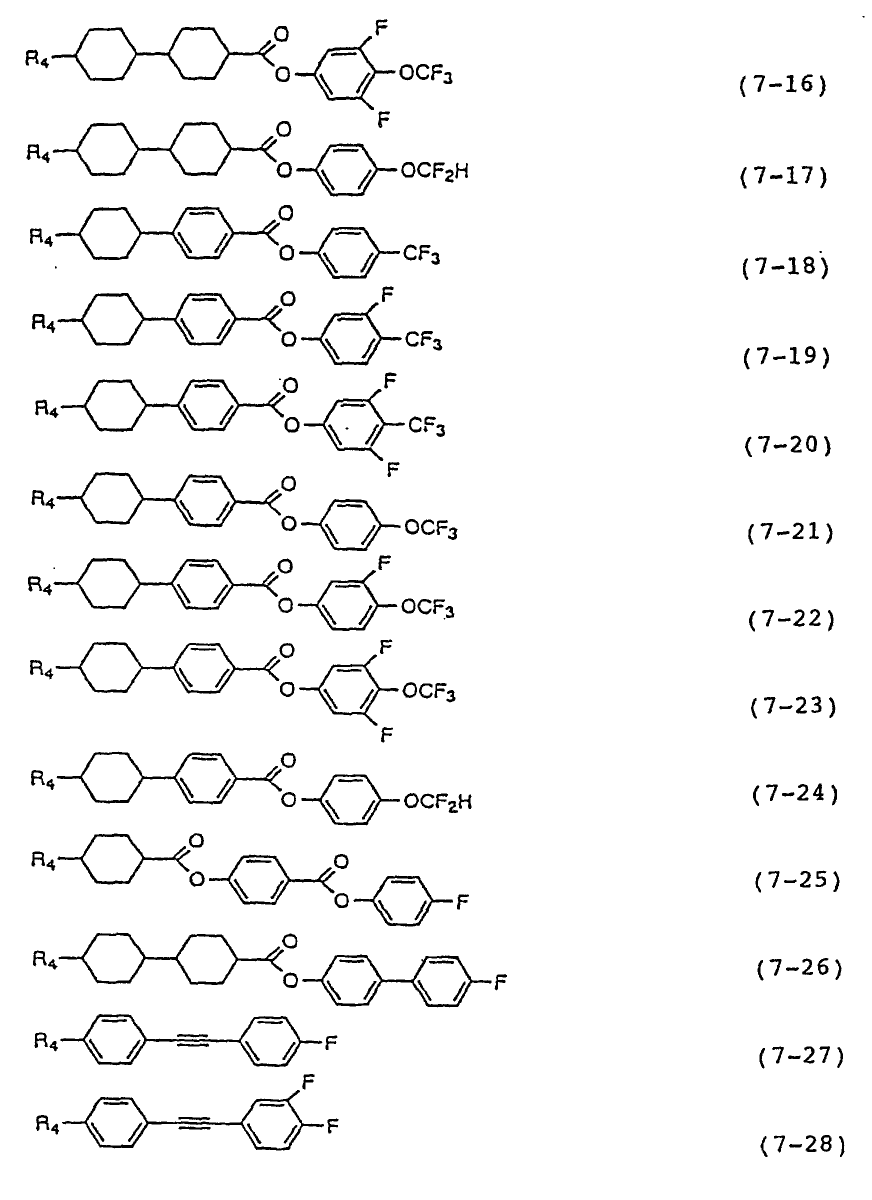 (wherein R₂, R₃, and R₄ have the same meanings as in the general formulae (5) to (7) shown above.)

The compounds expressed by one of the general formulae (5) to (7) have a positive large dielectric anisotropy and are used as components for lowering the threshold voltage of the liquid crystal compositions. Further, these compounds are used for the purposes of adjusting viscosity and adjusting optical anisotropy, and widening the temperature range of liquid crystal phase, and further for the purpose of improving the steepness of voltage - transmittance property.

Preferred examples of the compounds expressed by the general formulae (8) or (9) include compounds expressed by one of the general formulae (8-1) to (8-8) and general formulae (9-1) to (9-12) as follows: 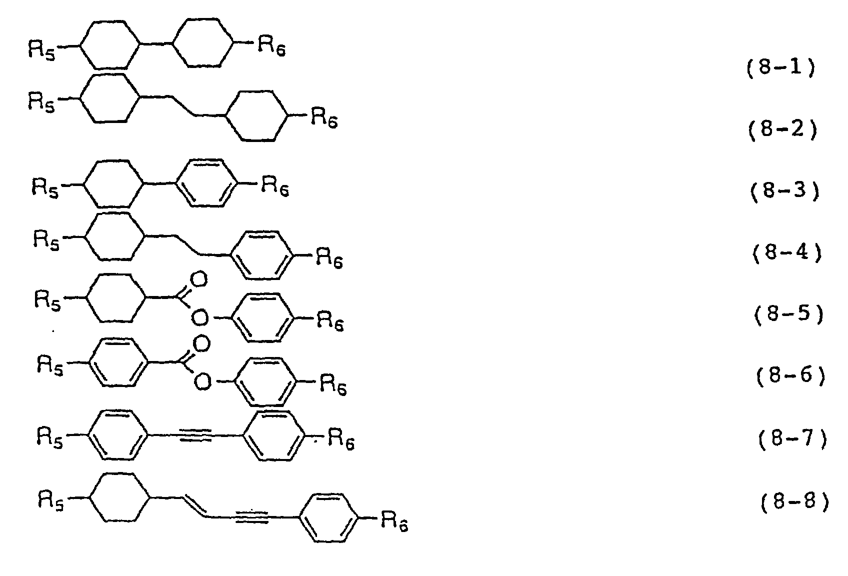 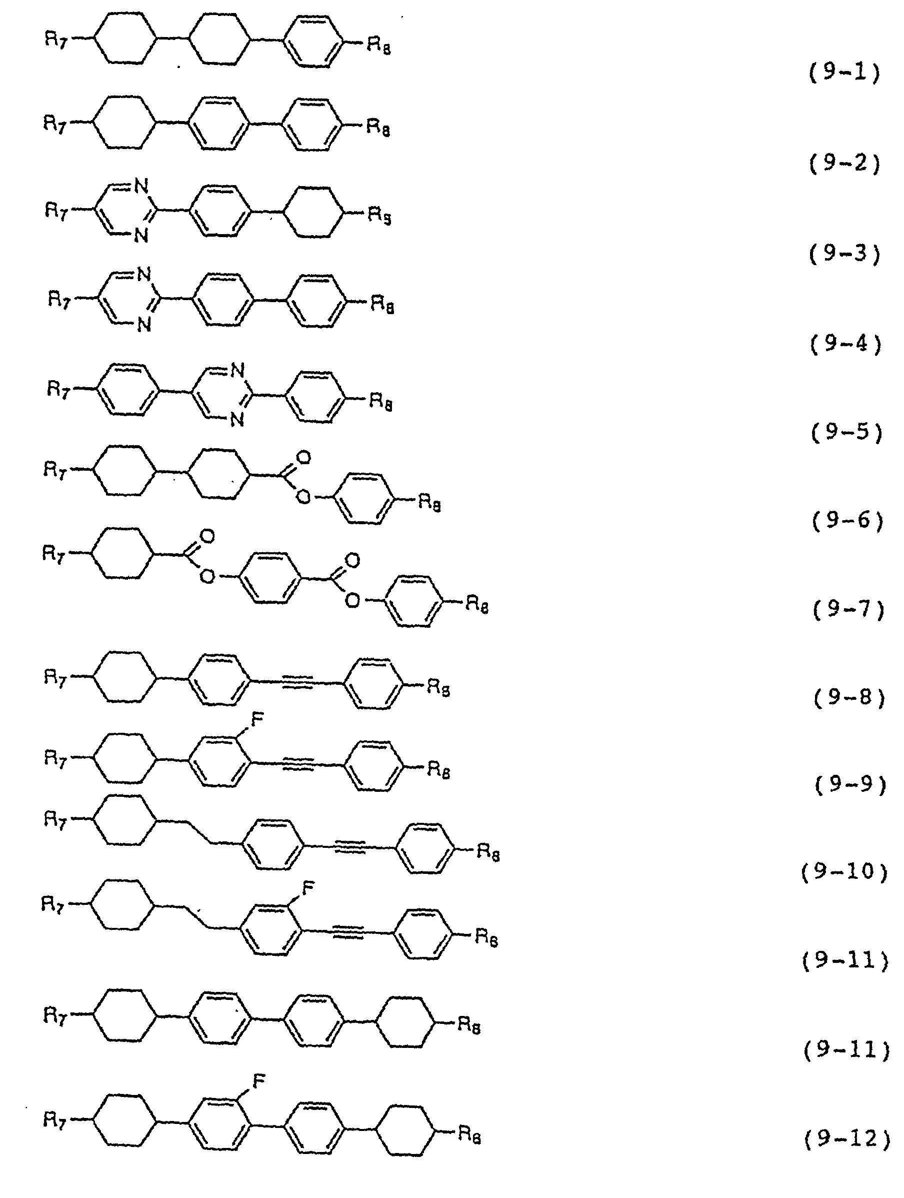 (wherein R₅ and R₆ have the same meanings as in the general formulae (8) to (9).)

The compounds expressed by the general formulae (8) or (9) have a negative or slightly positive dielectric anisotropy. The compounds expressed by the general formula (8) are used mainly for the purposes of lowering the viscosity of the liquid crystal compositions and/or the purpose of adjusting the optical anisotropy thereof. Further, the compounds of the general formula (9) are used for the purpose of widening the temperature range of the nematic phase of the liquid crystal compositions by raising the clearing point and/or the purpose of controlling the optical anisotropy thereof.

The compounds of one of the general formulae (5) to (9) are particularly useful for preparation of liquid crystal compositions for STN display mode and ordinary TN display mode.

Any of the compounds represented by the general formulas (5) to (9) can be used in any amount in the range of 1 to 99% by weight, preferably in an amount of 10 to 97% by weight, and more desirably in an amount of 40 to 95% by weight when liquid crystal compositions for ordinary TN display mode or STN display mode is produced. In this preparation, any of the compounds represented by the formulae (2) to (4) may be used in combination.

The liquid crystal compositions of the present invention can be produced in any conventional methods. In general, a method of dissolving various components each other at a high temperature is used. Alternatively, the compositions may be produced by dissolving the components in an organic solvent , mixing them, and then distilling off the solvent under a reduced pressure. By adding one or more known additives, modification or optimization of the compositions depending on intended uses can further be conducted.

The conventionally used additives include chiral dopant for inducing a helical structure of liquid crystals to adjust a necessary twist angle and to prevent reverse twist. Further, the liquid crystal compositions can be used as those for guest-host (GH) mode by adding a dichroic dye such as merocyanine type, styryl type, azo type, azomethine type, azoxy type, quinophthalone type, anthraquinone type, and tetrazine type.

By the use of the liquid crystal compositions of the present invention for TFT liquid crystal display devices, steepness of the voltage-transmittance characteristic and the viewing angle can be improved. In addition, the compounds of the general formula (1) have a low viscosity and thus the response speed of liquid crystal display devices can significantly be improved by using the compound. The liquid crystal compositions of the present invention can also be used for polymer dispersed liquid crystal display devices (PDLCD) represented by NCAP prepared by micro-encapsulation of nematic liquid crystal, or represented by polymer network liquid crystal display devices (PNLCD) prepared by forming a three-dimensionanetwork polymers in a liquid crystal; or used as liquid crystal compositions for electrically controlled birefringence (ECB) mode or dynamic scattering (DS) mode.

As nematic liquid crystal compositions comprising the liquid crystal compounds of the present invention expressed by the general formula (1), the following composition examples (Composition Examples 3, 4, 6, 8-12 and 14) can be mentioned. The compounds in the Composition Examples are expressed in terms of abbreviations according to the definition shown in the following Table.

Composition Examples 1, 2, 5, 7 and 13 are Comparative Examples.

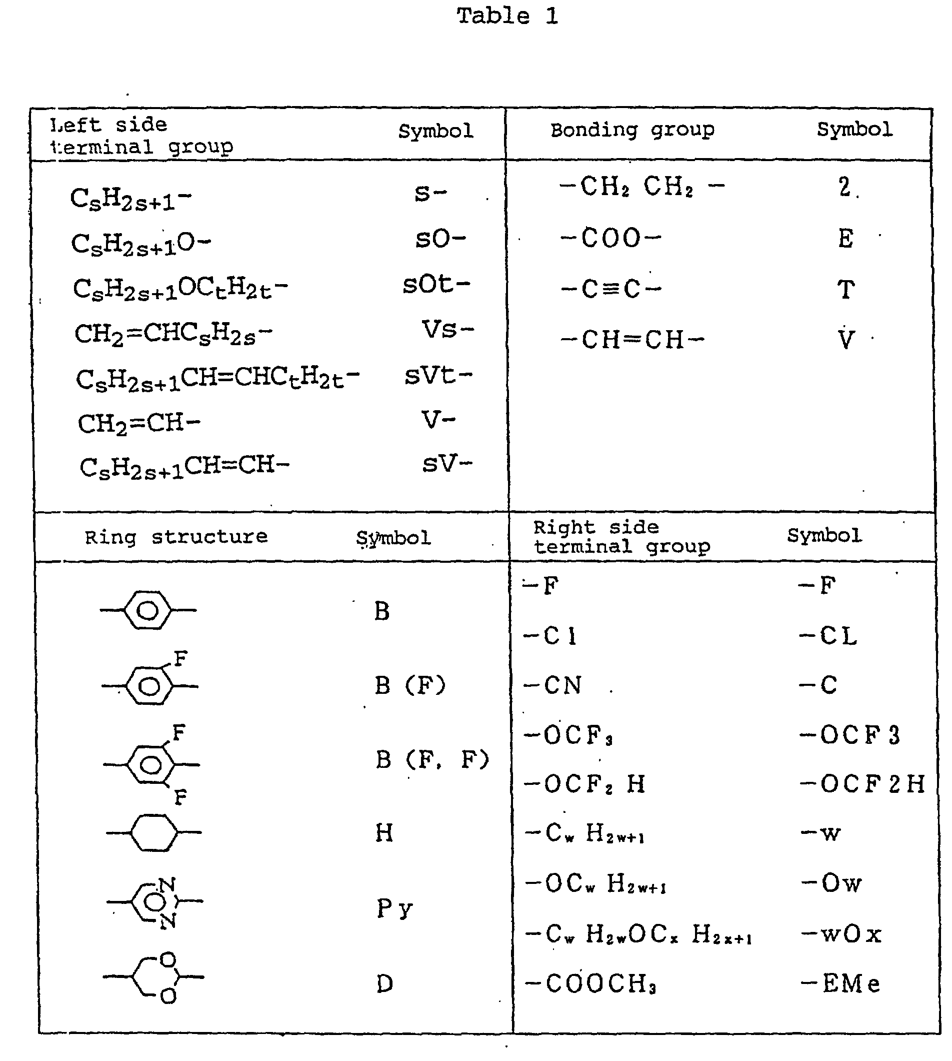

In Table 1, s, t, w, and x are an integer of 1 or more.

### Composition Example 1 (Comparative Example)

| | |
|---|---|
| 3-HVHVH-3 | 7.0% |
| 1V2-BEB (F, F) -C | 8.0% |
| 3-HB-C | 24.0% |
| 3-HB-O2 | 9.0% |
| 3-HH-4 | 10.0% |
| 3-HH-5 | 5.0% |
| 3-HHB-1 | 10.0% |
| 3-HHB-3 | 5.0% |
| 3-H2BTB-2 | 7.0% |
| 3-H2BTB-3 | 6.0% |
| 3-HB(F)TB-2 | 5.0% |
| 3-HB(F)TB-3 | 4.0% |

### Composition Example 2 (Comparative Example)

| | |
|---|---|
| 3-HVHVH-2 | 5.0% |
| 3-HVHVH-3 | 5.0% |
| 3O1-BEB(F)-C | 11.0% |
| 1V2-HB-C | 11.0% |
| 3-HB-O2 | 5.0% |
| 2-BTB-O1 | 6.0% |
| 3-BTB-O1 | 6.0% |
| 4-BTB-O1 | 6.0% |
| 4-BTB-O2 | 6.0% |
| 5-BTB-O1 | 6.0% |
| 3-H2BTB-2 | 2.0% |
| 3-H2BTB-3 | 3.0% |
| 3-HB(F)TB-2 | 6.0% |
| 3-HB(F)TB-3 | 6.0% |
| 2-PyBH-3 | 4.0% |
| 3-PyBH-3 | 4.0% |
| 3-PyBB-2 | 4.0% |
| 3-HEBEB-1 | 2.0% |
| 3-HEBEB-F | 2.0% |

### Composition Example 3

| | |
|---|---|
| 3-HVHHVH-5 | 5.0% |
| 2-BB-C | 8.0% |
| 4-BB-C | 6.0% |
| 2-HB-C | 10.0% |
| 3-HB-C | 13.0% |
| 3-HHB-F | 5.0% |
| 2-HHB-C | 4.0% |
| 3-HHB-C | 6.0% |
| 5-PyB-F | 6.0% |
| 3-PyBB-F | 6.0% |
| 3-BTB-1 | 5.0% |
| 2-HHB-1 | 6.0% |
| 3-HHB-1 | 5.0% |
| 3-HHB-3 | 10.0% |
| 3-HHB-O1 | 5.0% |

### Composition Example 4

| | |
|---|---|
| 2-HVHVHH-1 | 3.0% |
| 3-HVHVHB-2 | 3.0% |
| 3-DB-C | 10.0% |
| 4-DB-C | 12.0% |
| 5-DB-C | 8.0% |
| 2-BEB-C | 10.0% |
| 5-PyB(F)-F | 10.0% |
| 2-PyB-O2 | 4.0% |
| 2-PyB-2 | 2.0% |
| 3-PyB-2 | 2.0% |
| 3-HEB-O4 | 5.0% |
| 4-HEB-O2 | 5.0% |
| 3-HEB-O2 | 4.0% |
| 1O-BEB-2 | 3.0% |
| 5-HEB-1 | 3.0% |
| 4-HEB-4 | 5.0% |
| 2-PyBH-3 | 5.0% |
| 3-HHEBB-C | 3.0% |
| 3-HBEBB-C | 3.0% |

### Composition Example 5 (Comparative Example)

| | |
|---|---|
| 3-HVHVH-1 | 3.0% |
| 3-HVHVH-3 | 4.0% |
| V2-HB-C | 10.0% |
| 1V2-HB-C | 8.0% |
| 3-HB-C | 14.0% |
| 1O1-HB-C | 6.0% |
| 2O1-HB-C | 6.0% |
| 2-HHB-C | 5.0% |
| 3-HHB-C | 5.0% |
| 3-HH-4 | 10.0% |
| 1O1-HH-5 | 5.0% |
| 3-HH-EMe | 3.0% |
| 2-BTB-O1 | 11.0% |
| 3-HHB-1 | 5.0% |
| 3-HHB-3 | 5.0% |

### Composition Example 6

| | |
|---|---|
| 3-HVHVH-3 | 4.0% |
| 3-HVHHVH-5 | 4.0% |
| 3-HB(F)-C | 4.0% |
| 2O1-BEB(F)-C | 4.0 % |
| 3O1-BEB(F)-C | 12.0% |
| 1V2-BEB(F,F)-C | 10.0% |
| 3-HHB(F)-C | 5.0% |
| 3-HHEB-F | 5.0% |
| 5-HHEB-F | 5.0% |
| 3-HBEB-F | 6.0% |
| 3-HBB-F | 3.0% |
| 3-HB-O2 | 10.0% |
| 3-HH-4 | 5.0% |
| 3-HHB-1 | 6.0% |
| 3-HHB-O1 | 4.0% |
| 3-H2BTB-2 | 4.0% |
| 3-H2BTB-3 | 4.0% |
| 3-HB(F)TB-2 | 5.0% |

### Composition Example 7 (Comparative Example)

| | |
|---|---|
| 3-HVHVH-4 | 5.0 % |
| 5-HVHVH-3 | 5.0% |
| 3-H2HB(F,F)-F | 10.0% |
| 5-H2HB(F,F)-F | 8.0% |
| 3-HHB(F,F)-F | 10.0% |
| 4-HHB(F,F)-F | 6.0% |
| 3-HH2B(F,F)-F | 12.0% |
| 5-HH2B(F,F)-F | 8.0% |
| 3-HBB(F,F)-F | 14.0% |
| 5-HBB(F,F)-F | 10.0% |
| 3-HHEB(F,F)-F | 6.0% |
| 3-HBEB(F,F)-F | 2.0% |
| 3-HHBB(F,F)-F | 2.0% |
| 3-HH2BB(F,F)-F | 2.0% |

### Composition Example 8

| | |
|---|---|
| 3-HVHVH-3 | 6.0% |
| 3-HVHHVH-5 | 4.0% |
| 7-HB(F)-F | 7.0% |
| 5-H2B(F)-F | 4.0% |
| 2-HHB(F)-F | 6.0% |
| 3-HHB(F)-F | 6.0% |
| 5-HHB(F)-F | 6.0% |
| 2-H2HB(F)-F | 4.0% |
| 3-H2HB(F)-F | 2.0% |
| 5-H2HB(F)-F | 4.0% |
| 3-H2HB(F,F)-F | 6.0% |
| 4-H2HB(F,F)-F | 5.0% |
| 5-H2HB(F,F)-F | 5.0% |
| 3-HHB(F,F)-F | 8.0% |
| 3-HH2B(F,F)-F | 8.0% |
| 5-HH2B(F,F)-F | 7.0% |
| 3-HEB-F | 6.0% |
| 5-HHEBB-F | 3.0% |
| 1O1-HBBH-3 | 3.0% |

### Composition Example 9

| | |
|---|---|
| 3-HVHVHH-5 | 4.0% |
| 3-HVHVHB-3 | 4.0% |
| 5-HB-CL | 4.0% |
| 7-HB(F,F)-F | 6.0% |
| 3-HBB(F)-F | 10.0% |
| 5-HBB(F)-F | 10.0% |
| 3-H2BB(F)-F | 10.0% |
| 2-HHB-CL | 5.0% |
| 4-HHB-CL | 10.0% |
| 5-HHB-CL | 5.0% |
| 3-HBB(F,F)-F | 12.0% |
| 5-HBB(F,F)-F | 5.0% |
| 3-H2BB(F,F)-F | 5.0% |
| 3-HB(F)VB-2 | 5.0% |
| 3-HB(F)VB-3 | 5.0% |

### Composition Example 10

| | |
|---|---|
| 2-HVHVHB-3 | 5.0% |
| 7-HB-F | 7.0% |
| 3-HH-O1 | 7.0% |
| 3-HB-O1 | 6.0% |
| 3-HHB-OCF2H | 3.0% |
| 5-HHB-OCF2H | 4.0% |
| 3-HHB(F,F)-OCF2H | 10.0% |
| 5-HHB(F,F)-OCF2H | 8.0% |
| 2-HHB-OCF3 | 6.0% |
| 3-HHB-OCF3 | 7.0% |
| 5-HHB-OCF 3 | 6.0% |
| 3-HH2B-OCF3 | 6.0% |
| 3-HH2B(F)-F | 7.0% |
| 5-HH2B(F)-F | 9.0% |
| 3-HHEB(F)-F | 6.0% |
| 5-HB(F)BH-3 | 3.0% |

### Composition Example 11

| | |
|---|---|
| 3-HVHVH-2 | 6.0% |
| 3-HVHHVH-3 | 4.0% |
| V-HB-C | 10.0% |
| 1V-HB-C | 5.0% |
| 3-BB-C | 5.0% |
| 5-BB-C | 5.0% |
| 2-HB(F)-C | 5.0% |
| 4-BB-3 | 3.0% |
| 3-H2B-O2 | 8.0% |
| 5-H2B-O2 | 5.0% |
| 3-BEB-C | 5.0% |
| 5-HEB-O1 | 5.0% |
| 5-HEB-O3 | 8.0% |
| 5-BBB-C | 3.0% |
| 4-BPyB-C | 4.0% |
| 4-BPyB-5 | 4.0% |
| 5-HB2B-4 | 3.0% |
| 5-HBB2B-3 | 2.0% |
| 1V-HH-1O1 | 5.0% |
| 1V2-HBB-3 | 5.0% |

### Composition Example 12

| | |
|---|---|
| 2-HVHVHH-2 | 3.0 % |
| 3-HVHVHH-2 | 3.0% |
| 5-HVHVHB-2 | 4.0% |
| 4-HEB(F)-F | 10.0% |
| 5-HEB(F)-F | 10.0% |
| 2-BEB(F)-C | 5.0% |
| 3-BEB(F)-C | 5.0% |
| 4-BEB(F)-C | 8.0% |
| 5-BEB(F)-C | 8.0% |
| 1O3-HB(F)-C | 6.0% |
| 3-HHEB(F)-F | 5.0% |
| 5-HHEB(F)-F | 5.0% |
| 2-HBEB(F)-C | 5.0% |
| 3-HBEB(F)-C | 5.0% |
| 5-HBEB(F)-C | 5.0% |
| 3-HBTB-2 | 5.0% |
| V2-HH-3 | 4.0% |
| V2-HHB-1 | 4.0% |

### Composition Example 13 (Comparative Example)

| | |
|---|---|
| V-HVHVH-2V | 3.0% |
| 2V-HVHVH-V2 | 4.0% |
| V2-HB-C | 10.0% |
| 1V2-HB-C | 8.0% |
| 3-HB-C | 14.0% |
| 1O1-HB-C | 6.0% |
| 2O1-HB-C | 6.0% |
| 2-HHB-C | 5.0% |
| 3-HHB-C | 5.0% |
| 3-HH-4 | 10.0% |
| 1O1-HH-5 | 5.0% |
| 3-HH-EMe | 3.0% |
| 2-BTB-O1 | 11.0% |
| 3-HHB-1 | 5.0% |
| 3-HHB-3 | 5.0% |

### Composition Example 14

| | |
|---|---|
| 1V-HVHVHH-2V1 | 4.0% |
| V-HVHVHB-5 | 4.0% |
| 5-HB-CL | 4.0% |
| 7-HB(F,F)-F | 6.0% |
| 3-HBB(F)-F | 10.0% |
| 5-HBB(F)-F | 10.0% |
| 3-H2BB(F)-F | 10.0% |
| 2-HHB-CL | 5.0% |
| 4-HHB-CL | 10.0% |
| 5-HHB-CL | 5.0% |
| 3-HBB(F,F)-F | 12.0% |
| 5-HBB(F,F)-F | 5.0% |
| 3-H2BB(F,F)-F | 5.0% |
| 3-HB(F)VB-2 | 5.0% |
| 3-HB(F)VB-3 | 5.0% |

Now, the present invention is described in more detail with reference to Examples. However, the scope of the present invention is by no means restricted by such specific Examples. In the Examples, the structures of the resulting compounds were confirmed by nuclear magnetic resonance spectrum (hereinafter abbreviated to ¹H-NMR) and mass spectrum (hereinafter abbreviated to MS). In the Examples, d is doublet; m, multiplet; and J, coupling constant. In MS, M⁺ is molecular ion peak, and numbers in the parentheses are ionic strength. Among the symbols exhibiting phase transition, Cr indicates crystal phase; S, smectic phase; N, nematic phase; and Iso, isotropic liquid phase. The unit of phase transition temperature in every case is °C.

### Example 1 (Comparative Example)

Preparation of trans-1,4-bis-((E)-2-(trans-4-propylcyclohexyl)ethenyl)cyclohexane (Compound expressed by the general formula (1) wherein A₁ and A₂ are single bond, and R and R' each are propyl group)

### First step

In 3.5 L of THF was suspended 367.7 g (763.7 mmol) of (trans-4-propylcyclohexyl)methyltriphenylphosphonium bromide, and cooled down to -50°C under a nitrogen gas stream. To this mixture was added 94.3 g (840.4 mmol) of t-BuOK, and stirred for 1 hour. Then, 1 L of THF solution containing 100.0 g (587.5 mmol) of methyl trans-4-formylcyclohexane carboxylate was added dropwise to the mixture over 30 minutes while maintaining a temperature lower than -50°C. After finishing of the dropping, the reaction temperature was raised gradually up to room temperature and the mixture was further stirred for 5 hours. From the reaction solution, the solvent was distilled off under a reduced pressure, 500 ml of water was added to the residue, and the product was extracted with 500 ml of toluene. The organic layer was washed thrice with 200 ml of water and dried over anhydrous magnesium sulfate, and the solvent was then distilled off under a reduced pressure. The residue was purified through silica gel column chromatography (eluent: heptane) to obtain 140.9 g of crude methyl trans-4-(2-(trans-4-propylcyclo-hexyl)ethenyl)cyclohexanecarboxylate.

### Second step

To 200 ml of THF was added 13.7 g (360.1 mmol) of lithium aluminum hydride on an ice bath under a nitrogen gas stream, and 1.5 L of THF solution containing 140.9 g (481.8 mmol) of the methyl trans-4-(2-(trans-4-propylcyclohexyl)-ethenyl)cyclohexanecarboxylate obtained in the first step was added dropwise thereto over 40 minutes while stirring. After finishing of the dropping, the mixture was stirred for 5 hours at room temperature, and 200 ml of 2 N sodium hydroxide aqueous solution was gradually added thereto on an ice bath to terminate the reaction. The reaction solution was heated at 50°C on a water bath, and when the suspended material formed precipitates, these were filtered off with Celite, and the solvent was distilled off under a reduced pressure from the filtrate to obtain 76.4 g of crude trans-4-(2-(trans-4-propylcyclohexyl)ethenyl)cyclohexane carbinol.

### Third step

To 800 ml of methylene chloride were added 68.5 g (317.8 mmol) of PCC and 68.5 g of silica gel on an ice bath wider a nitrogen gas stream, and 76.4 g (288.9 mmol) of the trans-4-(2-(trans-4-propylcyclohexyl)ethenyl)cyclohexane carbinol obtained in the second step was added while stirring, and the mixture was stirred on an ice bath for 1 hour and further at room temperature for 5 hours. Methylene chloride insolubles were removed from the reaction solution by filtration, and the solvent was then distilled off under a reduced pressure. The residue was purified through silica gel column chromatography (eluent: heptane) to obtain 44.0 g of crude trans-4-(2-(trans-4-propylcyclohexyl)ethenyl)-cyclohexane carbaldehyde.

### Fourth step

In 1 L of THF was suspended 105.0 g (218.1 mmol) of (trans-4-propylcyclohexyl)methyltriphenylphosphonium bromide, and cooled down to -50°C under a nitrogen gas stream. To this mixture was added dropwise 26.9 g (239.7 mmol) of t-BuOK, and stirred for 1 hour. Then, 450 ml of THF solution containing 44.0 g (167.7 mmol) of trans-4-(2-(trans-4-propylcyclohexyl)ethenyl)cyclohexane carbaldehyde obtained in the third step was added dropwise thereto over 20 minutes while maintaining a temperature of -50°C. After finishing of the dropping, the reaction temperature was raised gradually up to room temperature and the mixture was further stirred for 5 hours. From the reaction solution, the solvent was distilled off under a reduced pressure, 250 ml of water was added to the residue, and the product was extracted with 250 ml of toluene. The organic layer was washed thrice with 100 ml of water and dried over anhydrous magnesium sulfate, and the solvent was then distilled off under a reduced pressure. The residue was purified through silica gel column chromatography (eluent: heptane) to obtain 51.6 g of crude trans-1,4-bis-(2-(trans-4-propylcyclohexyl) ethenyl)cyclohexane.

### Fifth step

To 1.0 L of methylene chloride were added 92.5 g (536.1 mmol) of 3-chloroperbenzoic acid (mCPBA) and 185.2 g (1340.0 mmol) of potassium carbonate on an ice bath under a nitrogen gas stream, and 500 ml of methylene chloride solution containing 51.6 g (134.1 mmol) of trans-1,4-bis-(2-(trans-4-propylcyclohexyl)ethenyl)cyclohexane obtained in the fourth step was added dropwise thereto over 20 minutes. After finishing of the dropping, the mixture was stirred at room temperature for 5 hours and the reaction was terminated by adding 500 ml of saturated aqueous sodium of thiosulfate solution. The organic layer was recovered from the reaction solution, then washed twice with 500 ml of saturated aqueous solution of sodium bicarbonate and thrice with 500 ml of water, and dried over anhydrous sodium sulfate, and the solvent was distilled off under a reduced pressure. The residue was purified through silica gel column chromatography (eluent: a mixed solvent of ethyl acetate/heptane = 1/9) to obtain 39.1 g of crude trans-1,4-bis-(2-(trans-4-propylcyclo hexyl)epoxyethyl)cyclohexane.

### Sixth step

In 400 ml of toluene were dissolved 39.1 g (93.8 mmol) of the trans-1,4-bis-(2-(trans-4-propylcyclohexyl)-epoxyethyl)cyclohexane obtained in the fifth step and 126.7 g (300.2 mmol) of triphenylphosphine dibromide, and heated to reflux for 3 hours. From the reaction solution, the solvent was distilled off under a reduced pressure. The residue was purified through silica gel column chromatography (eluent: toluene) and further recrystallized from a mixed solvent of heptane/ethanol (5/1) to obtain 14.5 g of trans-1,4-bis-((E)-1,2-dibromo-2-(trans-4-propylcyclohexyl)ethyl)-cyclohexane.

### Seventh step

To 150 ml of toluene were added 14.5 g (20.6 mmol) of trans-1,4-bis-((E)-1,2-dibromo-2-(trans-4-propylcyclohexyl)ethyl)cyclohexane, 8.1 g (123.9 mmol) of zinc, and 150 ml of acetic acid, and stirred at room temperature for 12 hours. Insolubles were removed from the reaction solution by filtration, and 150 ml of water was added to the filtrate. The organic layer was recovered from this mixture, then washed thrice with 150 ml of saturated aqueous solution of sodium bicarbonate and thrice with 150 ml of water, and dried over anhydrous magnesium sulfate, and the solvent was distilled off under a reduced pressure. The residue was purified through silica gel column chromatography (eluent: a mixed solvent of ethyl acetate/heptane = 1/9) to obtain a crude product of trans-1,4-bis-((E)-2-(trans-4-propylcyclo-hexyl)ethenyl)cyclohexane. This crude product was re-crystallized from a mixed solvent of heptane/ethanol (1/2) to obtain 4.1 g of the subject compound (yield: 1.8 %). The results of measurement of various spectrum data indicated the structure of the object compound.

Phase transition temperature: Cr room temperature or less S_{B} 171.8 N 210.3 Iso
MS: m/e = 384 (M⁺, 48%), 299 (23), 189 (18), 135 (24), 81 (72), 67 (100).
NMR: Shown in the chart of Fig. 1.

### Example 2

Preparation of trans-4-((E)-2-(trans-4-propylcyclohexyl) ethenyl) -trans-4'-((E)-2-(trans-4-pentylcyclohexyl)ethenyl)bicyclohexane (Compound expressed by the general formula (1) wherein A₁ is trans-1,4-cyclohexylene group, A₂ is single bond, R is propyl group, and R' is pentyl group; Compound No. 5)

### First stage

In 2.5 L of THF was suspended 262.5 g (515.2 mmol) of (trans-4-pentylcyclohexyl)methyltriphenylphosphonium bromide, and cooled down to -50°C under a nitrogen gas stream. To the suspension was added 63.6 g (566.8 mmol) of t-BuOK and stirred for 1 hour. Then, 1 L of THF solution containing 100.0 g (396.3 mmol) of methyl trans-4-(trans-4-formylcylohexyl)cyclohexanecarboxylate was added dropwise to this mixture over 30 minutes while maintaining a temperature lower than -50°C. After finishing of the dropping, the reaction temperature was raised gradually up to room temperature and the mixture was further stirred for 5 hours. From the reaction solution, the solvent was distilled off under a reduced pressure, 500 ml of water was added to the residue, and the product was extracted with 500 ml of toluene. The organic layer was washed thrice with 200 ml of water and dried over anhydrous magnesium sulfate, and the solvent was then distilled off under a reduced pressure. The residue was purified through silica gel column chromatography (eluent: heptane) to obtain 132.4 g of crude methyl trans-4-(trans-4-(2-(trans-4-pentylcyclohexyl)-ethenyl)cyclohexyl)cyclohexanecarboxylate.

### Second step

To 200 ml of THF was added 9.4 g (247.7 mmol) of lithium aluminum hydride on an ice bath under a nitrogen gas stream, and 1.3 L of THF solution containing 132.4 g (328.8 mmol) of the methyl trans-4-(trans-4-(2-(trans-4-pentylcyclohexyl)ethenyl)cyclohexyl)cyclohexanecarboxylate obtained in the first step was added dropwise to this mixture over 40 minutes while stirring. The mixture was stirred for 5 hours at room temperature, and 300 ml of 2 N sodium hydroxide aqueous solution was gradually added to it on an ice bath to terminate the reaction. The reaction solution was heated at 50°C on a water bath, and when the suspended material formed precipitates, these were filtered off with Celite, and the solvent was distilled off under a reduced pressure from the filtrate to obtain 72.7 g (194.0 mmol) of crudee trans-4-(trans-4-(2-(trans-4-pentylcyclohexyl)ethenyl)cyclohexyl)cyclohexane carbinol.

### Third step

To 700 ml of methylene chloride were added 46.0 g (213.4 mmol) of PCC and 46.0 g of silica gel on an ice bath under a nitrogen gas stream and the mixture was stirred. Then, 72.7 g (194.0 mmol) of the trans-4-(trans-4-(2-(trans-4 -pentylcyclohexyl) ethenyl) cyclohexyl)cyclohexane carbinol obtained in the second step was added thereto, and the mixture was stirred on an ice bath for 1 hour and further at room temperature for 5 hours. Methylene chloride insolubles were removed from the reaction solution by filtration, and the solvent was then distilled off under a reduced pressure. The residue was purified through silica gel column chromatography (eluent: heptane) to obtain 40.5 g of crude trans-4-(trans-4-(2-(trans-4-pentylcyclohexyl)ethenyl)-cyclohexyl)cyclohexane carbaldehyde.

### Fourth step

In 700 ml of THF was suspended 68.0 g (141.2 mmol) of (trans-4-propylcyclohexyl)methyltriphenylphosphonium bromide, and cooled down to -50°C under a nitrogen gas stream. To this mixture was added 17.4 g (155.1 mmol) of t-BuOK and stirred for 1 hour. Then, 400 ml of THF solution containing 40.5 g (108.7 mmol) of the trans-4-(trans-4-(2-(trans-4-pentylcyclohexyl)ethenyl)cyclohexyl)cyclohexane carbaldehyde obtained in the third step was added dropwise to the mixture over 20 minutes while maintaining a temperature lower than -50°C. After finishing of the dropping, the reaction temperature was raised gradually up to room temperature and the mixture was further stirred for 5 hours. From the reaction solution, the solvent was distilled off under a reduced pressure, 200 ml of water was added to the residue, and the product was extracted with 200 ml of toluene. The organic layer was washed thrice with 100 ml of water and dried over anhydrous magnesium sulfate, and the solvent was then distilled off under a reduced pressure. The residue was purified through silica gel column chromatography (eluent: heptane) to obtain 44.1 g of crude trans-4-(2-(trans-4-propylcyclohexyl)ethenyl)-trans-4'-(2-(trans-4-pentylcyclohexyl)ethenyl)bicyclohexane.

### Fifth step

To 600 ml of methylene chloride were added 61.5 g (356.5 mmol) of mCPBA and 123.2 g (891.4 mmol) of potassium carbonate on an ice bath under a nitrogen gas stream, and 450 ml of methylene chloride solution containing 44.1 g (89.1 mmol) of the trans-4-(2-(trans-4-propylcyclohexyl)-ethenyl) -trans-4'-(2-(trans-4-pentylcyclohexyl)ethenyl)-bicyclohexane obtained in the fourth step was added dropwise thereto over 20 minutes. After finishing of the dropping, the mixture was stirred at room temperature for 5 hours, and the reaction was terminated by adding 450 ml of saturated aqueous solution of sodium thiosulfate. The organic layer was recovered from the reaction solution, then washed twice with 450 ml of saturated aqueous solution of sodium bicarbonate and thrice with 450 ml of water, and dried over anhydrous sodium sulfate, and the solvent was distilled off under a reduced pressure. The residue was purified through silica gel column chromatography (eluent: a mixed solvent of ethyl acetate/heptane = 1/9) to obtain 33.3 g of crude trans-4-(2-(trans-4-propylcyclohexyl)epoxyethyl)-trans-4'-(2-(trans-4-pentylcyclohexyl) epoxyethyl)bicyclohexane.

### Sixth step

In 350 ml of toluene were dissolved 33.3 g (63.2 mmol) of the trans-4-(2-(trans-4-propylcyclo-hexyl)epoxyethyl)-trans-4'-(2-(trans-4-pentylcyclohexyl)epoxyethyl)-bicyclohexane obtained in the fifth step and 85.4 g (202.3 mmol) of triphenylphosphine dibromide, and heated to reflux for 3 hours. From the reaction solution, the solvent was distilled off under a reduced pressure. The residue was purified through silica gel column chromatography (eluent: toluene) and further recrystallized from a mixed solvent of heptane/ethanol (5/1) to obtain 10.3 g of trans-4-((E)-1,2-dibromo-2-(trans-4-propylcyclohexyl)ethyl)-4'-((E)-1,2-dibromo-2-(trans-4-pentylcyclohexyl)ethyl)bicyclohexane.

### Seventh step

To 100 ml of toluene were added 10.3 g (12.6 mmol) of the trans-4-((E)-1,2-dibromo-2-(trans-4-propylcyclohexyl)-ethyl)-4'-((E)-1,2-dibromo-2-(trans-4-pentylcyclohexyl)-ethyl)bicyclohexane obtained in the sixth step, 4.9 g (74.9 mmol) of zinc, and 100 ml of acetic acid, and stirred at room temperature for 12 hours. After stirring, insolubles were removed from the reaction solution by filtration, and 100 ml of water was added to the filtrate. The organic layer was recovered from this mixture, then washed thrice with 100 ml of saturated aqueous solution of sodium bicarbonate and thrice with 100 ml of water, and dried over anhydrous magnesium sulfate, and the solvent was distilled off under a reduced pressure. The residue was purified through silica gel column chromatography (eluent: a mixed solvent of ethyl acetate/heptane = 1/9) to obtain crude trans-4-((E)-2-(trans-4-propylcyclohexyl)ethenyl)-trans-4'-((E)-2-(trans-4-pentylcyclohexyl)ethenyl)bicyclohexane. This crude product was recrystallized from a mixed solvent of heptane/ethanol (1/2) to obtain 3.0 g of the subject compound (yield: 1.5 %).
MS: m/e = 494 (M⁺)

### Example 3

Preparation of trans-1- ((E) -2-(trans-4-propylcyclo-hexyl)ethenyl)-4-((E)-2-(trans-4-(4-pentylphenyl)cyclo-hexyl)ethenyl)cyclohexane (Compound expressed by the general formula (1) wherein A₁ is single bond, A₂ is phenylene group, R is propyl group, and R' is pentyl groups; Compound No. 21)

### First step

In 4.5 L of THF was suspended 447.3 g (763.8 mmol) of (trans-4-(4-pentylphenyl)cyclohexyl)methyltriphenyl- phospho nium bromide, and cooled down to -50°C under a nitrogen gas stream. Next, 94.3 g (840.4 mmol) of t-BuOK was added to this mixture and stirred for 1 hour. Then, 1 L of THF solution containing 100.0 g (587.5 mmol) of methyl trans-4-formylcylohexanecarboxylate was added dropwise to the mixture over 30 minutes while maintaining a temperature lower than -50°C. After finishing of the dropping, the reaction temperature was raised gradually up to room temperature and the mixture was further stirred for 5 hours. From the reaction solution, the solvent was distilled off under a reduced pressure, 500 ml of water was added to the residue, and the product was extracted with 500 ml of toluene. The organic layer was washed thrice with 200 ml of water and dried over anhydrous magnesium sulfate, and the solvent was then distilled off under a reduced pressure. The residue was purified through silica gel column chromatography (eluent: heptane) to obtain 184.1 g of crude methyl trans-4- (2- (trans-4- (4-pentylphenyl) cyclohexyl)-ethenyl) cyclohexanecarboxylate.

### Second step

To 100 ml of THF was added 13.2 g (347.8 mmol) of lithium aluminum hydride on an ice bath under a nitrogen gas stream, and 1.8 L of THF solution containing 184.1 g (464.2 mmol) of the methyl trans-4-(2-(trans-4-(4-pentylphenyl)-cyclohexyl)ethenyl)cyclohexanecarboxylate obtained in the first step was added dropwise to this mixture over 40 minutes under stirring. After finishing of the dropping, the mixture was stirred for 5 hours at room temperature, and 200 ml of 2 N sodium hydroxide aqueous solution was gradually added thereto on an ice bath to terminate the reaction. The reaction solution was heated to 50°C on a water bath, and when the suspended material formed precipitates, these were filtered off with Celite, and the solvent was distilled off under a reduced pressure from the filtrate to obtain 106.1 g of crude trans-4-(2-(trans-(4-pentylphenyl)- cyclohexyl)ethenyl)cyclohexane carbinol.

### Third step

To 1.0 L of methylene chloride were added 68.2 g (316.4 mmol) of PCC and 68.2 g silica gel on an ice bath under a nitrogen gas stream, and the mixture was stirred. Then, 106.1 g (287.8 mmol) of the trans-4-(2-(trans-(4-pentylphenyl)cyclohexyl)ethenyl)cyclohexane carbinol obtained in the second step was added thereto, and the mixture was stirred on an ice bath for 1 hour and further at room temperature for 5 hours. Methylene chloride insolubles were removed from the reaction solution by filtration, and the solvent was then distilled off under a reduced pressure. The residue was subjected to silica gel column chromatography (eluent: heptane) to obtain 62.2 g of crude trans-4-(2-(trans-4-(4-pentylphenyl)cyclohexyl)ethenyl)cyclohexane carbaldehyde.

### Fourth step

In 1 L of THF was suspended 106.2 g (220.6 mmol) of (trans-4-propylcyclohexyl)methyltriphenylphosphonium bromide, and cooled down to -50°C under a nitrogen gas stream. Then, 27.2 g (242.4 mmol) of t-BuOK was added to this mixture and stirred for 1 hour. To this mixture was added dropwise 450 ml of THF solution containing 62.2 g (169.7 mmol) of the trans-4-(2-(trans-4-(4-pentylphenyl)-cyclohexyl)ethenyl)cyclohexane carbaldehyde obtained in the third step over 20 minutes while maintaining a temperature lower than -50°C. After finishing of the dropping, the reaction temperature was raised gradually up to room temperature and the mixture was further stirred for 5 hours. From the reaction solution, the solvent was distilled off under a reduced pressure, 250 ml of water was added to the residue, and the product was extracted with 250 ml of toluene. The organic layer was washed thrice with 100 ml of water and dried over anhydrous magnesium sulfate, and the solvent was then distilled off under a reduced pressure. The residue was purified through silica gel column chromatography (eluent: heptane) to obtain 64.7 g of crude trans-1-(2-(trans-4-propylcyclohexyl)ethenyl)-4-(2-(trans-4-(4-pentylphenyl)cyclohexyl)ethenyl)cyclohexane.

### Fifth step

To 1.0 L of methylene chloride were added 93.0 g (529.8 mmol) of mCPBA and 183.1 g (1324.8 mmol) of potassium carbonate in an ice bath under a nitrogen gas stream, and 500 ml of methylene chloride solution containing 64.7 g (132.4 mmol) of the trans-1-(2-(trans-4-propylcyclohexyl)-ethenyl)-4-(2-(trans-4-(4-pentylphenyl)cyclohexyl)ethenyl)-cyclohexane obtained in the fourth step was added dropwise thereto over 20 minutes. After finishing of the dropping, the mixture was stirred at room temperature for 5 hours, and the reaction was terminated by adding 500 ml of saturated aqueous solution of sodium thiosulfate. The organic layer was recovered from the reaction solution, then washed twice with 500 ml of saturated aqueous solution of sodium bicarbonate and thrice with 500 ml of water, and dried over anhydrous sodium sulfate, and the solvent was distilled off under a reduced pressure. The residue was purified through silica gel column chromatography (eluent: a mixed solvent of ethyl acetate/heptane = 1/9) to obtain 50.3 g of crude trans-1- (2-(trans-4-propylcyclohexyl)epoxyethyl)-4-(2-(trans-4-(4-pentylphenyl)cyclohexyl)epoxyethyl)cyclohexane.

### Sixth step

In 500 ml of toluene were dissolved 50.3 g (96.6 mmol) of the trans-1-(2-(trans-4-propyl-cyclohexyl)epoxyethyl)-4-(2-(trans-4-(4-pentylphenyl)cyclohexyl)epoxyethyl)-cyclohexane obtained in the fifth step and 130.5 g (309.2 mmol) of triphenylphosphine dibromide, and heated to reflux for 3 hours. From the reaction solution, the solvent was distilled off under a reduced pressure, and the residue was purified through silica gel column chromatography (eluent: toluene) and then re-crystallized from a mixed solvent of heptane/ethanol (5/1) to obtain 16.4 g of trans-1-((E)-1,2-dibromo-2-(trans-4-propylcyclohexyl)ethyl)-4-((E)-1,2-dibromo-2-(trans-4-(4-pentylphenyl)cyclohexyl)ethyl)-cyclohexane.

### Seventh step

To 150 ml of toluene were added 16.4 g (20.3 mmol) of the trans-1-((E) -1,2-dibromo-2-(trans-4-propylcyclohexyl)-ethyl)-4-((E)-1,2-dibromo-2-(trans-4-(4-pentylphenyl)-cyclohexyl)ethyl)cyclohexane obtained in the sixth step, 8.0 g (122.4 mmol) of zinc, and 150 ml of acetic acid, and stirred at room temperature for 12 hours. After stirring, insolubles were removed from the reaction solution by filtration, and 150 ml of water was added to the filtrate. The organic layer was recovered, then washed thrice with 150 ml of saturated aqueous solution of sodium bicarbonate and thrice with 150 ml of water, and dried over anhydrous magnesium sulfate, and the solvent was distilled off under a reduced pressure. The residue was purified through silica gel column chromatography (eluent: a mixed solvent of ethyl acetate/heptane = 1/9) to obtain a crude product of trans-1-((E)-2-(trans-4-propylcyclohexyl)ethenyl)-4-((E)-2-(trans-4-(4-pentylphenyl)cyclohexyl) ethenyl)cyclohexane. This crude product was recrystallized from a mixed solvent of heptane /ethanol (1/2) to obtain 5.0 g of the subject compound (yield: 1.8 %).
MS: m/e = 488 (M+)

### Example 4 (Use Example 1 (Comparative Example)

The clearing point (Cp.) of a nematic liquid crystal composition comprising the following compounds in the amount shown below was 52.3°C:
4-(trans-4-propylcyclohexyl)benzonitrile 30% (by weight; this applies hereinafter);
4-(trans-4-pentylcyclohexyl)benzonitrile 40 %; and
4-(trans-4-heptylcyclohexyl)benzonitrile 30 %.

When this liquid crystal composition was filled in a TN cell (twist nematic cell) having a cell thickness of 9 µm, the composition exhibited the following physical properties:
driving threshold voltage (Vₜₕ) 1.60 V
dielectric anisotropy (Δε) 10.7
optical anisotropy (Δn) 0.119
viscosity at 20°C (η₂₀) 21.4 mPa·s.

This liquid crystal composition was used as mother liquid crystal A, and 85 parts by weight of this mother liquid crystal A was mixed with 15 parts by weight of the trans-1,4-bis((E)-2-(trans-4-propylcyclohexyl)etheny l)cyclohexane (Compound No. X) shown in Example 1 (Comparative) to obtain a new liquid crystal composition. Physical properties of this composition were then determined. As the result, Cp. was 83.2°C; Vₜₕ, 1.99 V; Δε, 9.5; Δn, 0.137; and η₂₀, 19.3 mPa·s. Although this composition was left in a freezer at -20°C for 25 days, neither separation of crystals nor development of smectic phase was observed.

### Example 5 (Comparative Example)

As a compound to be compared with the compounds of the present invention, Compound (b) described in the section of BACKGROUND ART was actually synthesized according to the method described in the publication mentioned in that section. A composition which was obtained by adding 15 parts by weight of Compound (b) to 85 parts by weight of the mother liquid crystal A described in Example 4 (Comparative) was measured for its physical properties. The results of the measurement of its viscosity and mutual solubility together with the results in Example 4 (Comparative) are shown in Table 2.

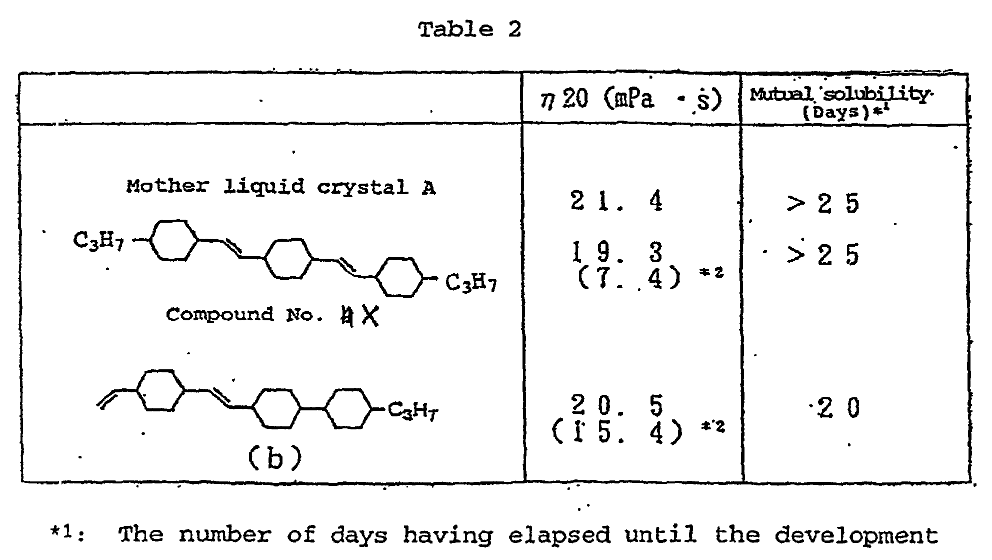

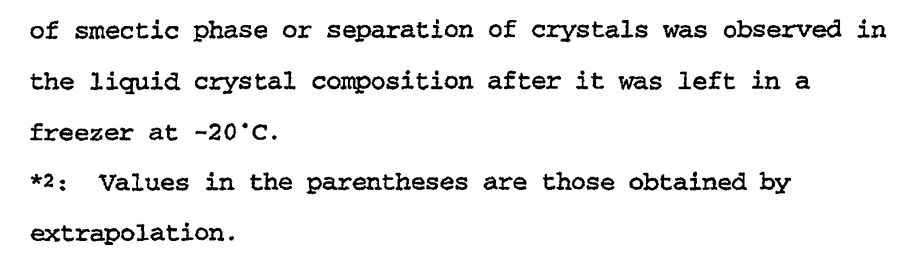

### INDUSTRIAL APPLICABILITY

Any compounds of the present invention expressed by the general formula (1), that is, 4-ring liquid crystalline compounds having two trans-1,2-ethenylene groups having a ring therebetween in the center portion of the molecule, exhibit a liquid crystal phase over a wide temperature range, have a low viscosity, high optical anisotropy, and large elastic constant ratio K₃₃/K₁₁, are readily mixed with various other liquid crystal materials even at low temperatures, and are considerably excellent as components of nematic liquid crystal compositions suitable for STN type display mode.

In addition, the compounds of the present invention have lower viscosity than that of conventional compounds, particularly than that of the compound described in Laid-Open Japanese Patent Application No. Sho 61-215336 and also have remarkably excellent mutual solubility at low temperatures.

Accordingly, when the compounds of the present invention are used as component of liquid crystal compositions, new liquid crystal compositions having desired physical properties, in addition to the characteristics of excellent mutual solubility with other liquid crystal materials, can be provided by selecting suitable 6-membered rings, substituents, and/or bonding groups as the molecule constituting elements.

## Claims

1. A liquid crystalline compound expressed by the general formula (1) : 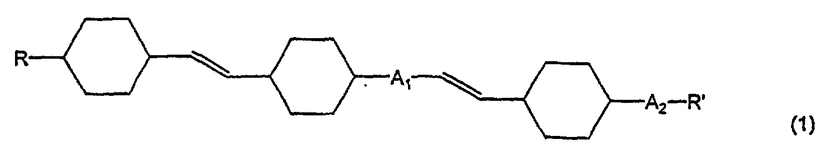 wherein R is a C₁-C₁₀ alkyl group or a C₂-C₁₀ alkenyl group; R' is a C₁-C₁₀ alkyl group, a C₂-C₁₀ alkenyl group, F, Cl, CN, OCF₃, CF₃, OCF₂H, CF₂H, or CFH₂; A₁ and A₂ are independently trans-1,4-cyclohexylene group, 1,4-phenylene group in which one or more hydrogen atoms on the 6-membered ring may be replaced by a halogen atom, or single bond, provided that A₁ and A₂ are selected such that the number of rings in the compound of formula (1) is four; and each of the elements constituting the compound may be replaced by an isotope thereof.

2. The liquid crystalline compound according to claim 1 wherein A₁ is trans-1,4-cyclohexylene group and A₂ is single bond in the general formula (1).

3. The liquid crystalline compound according to claim 1 wherein A₁ is single bond and A₂ is trans-1,4-cyclohexylene group or 1,4-phenylene group in the general formula (1).

4. A liquid crystal composition comprising at least two components and comprising at least one liquid crystalline compound defined in any one of claims 1 to 3.

5. A liquid crystal composition according to claim 4, further comprising as a second component, at least one compound selected from the group consisting of the compounds expressed by one of the general formulae (2), (3), and (4): 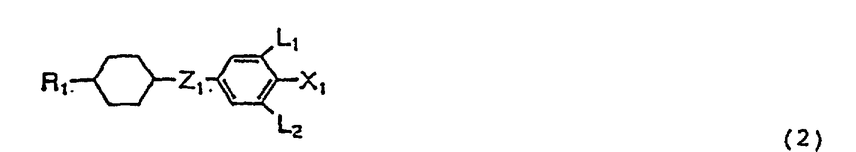 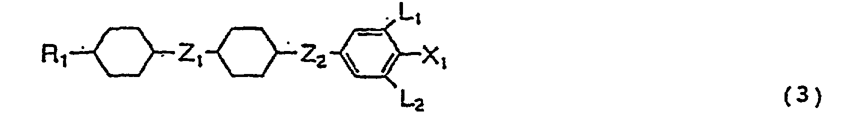 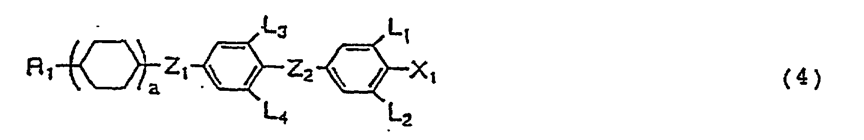 wherein R₁ is a C₁-C₁₀ alkyl group; X₁ is F, Cl, OCF₃, OCF₂H, CF₃, CF₂H, or CFH₂; L₁, L₂, L₃, and L₄ are independently H or F; Z₁ and Z₂ are independently -(CH₂)₂-, -CH=CH-, or single bond; and a is 1 or 2.

6. A liquid crystal composition according to claim 4, further comprising, as a second component, at least one compound selected from the group consisting of the compounds expressed by any one of the general formulae (5), (6), (7), (8), and (9): 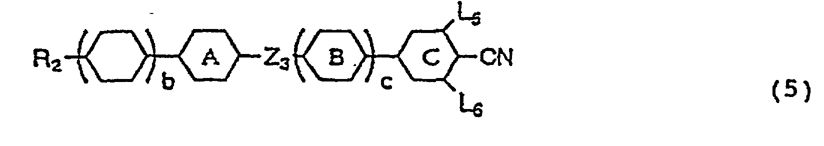 wherein R₂ is F, a C₁-C₁₀ alkyl group or C₂-C₁₀ alkenyl group, any not adjacent methylene group in said alkyl group or alkenyl group may be replaced by oxygen atom; ring A is trans-1,4-cylcohexylene group, 1,4-phenylene group, pyrimidine-2,5-diyl group, or 1,3-dioxane-2,5-diyl group; ring B is trans-1,4-cyclohexylene group, 1,4-phenylene group, or pyrimidine-2,5-diyl group; ring C is trans-1,4-cyclohexylene group or 1,4-phenylene group; Z₃ is -(CH₂)₂-, -COO- or single bond; L₅ and L₆ are independently H or F; and b and c are independently 0 or 1; 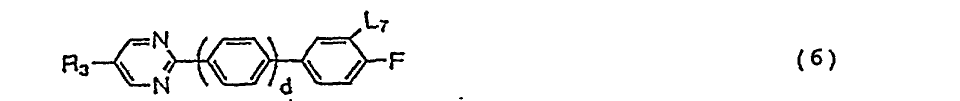 wherein R₃ is a C₁-C₁₀ alkyl group; L₇ is H or F; and d is 0 or 1; 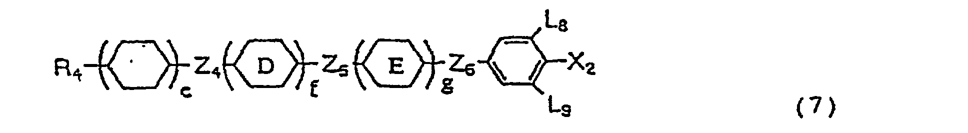 wherein R₄ is a C₁-C₁₀ alkyl group; rings D and E are independently trans-1,4-cyclohexylene group or 1,4-phenylene group; Z₄ and Z₅ are independently -COO- or single bond; Z₆ is -COO- or -C≡C-; L₈ and L₉ are independently H or F; X₂ is F, OCF₃, OCF₂H, CF₃, CF₂H, or CFH₂; and e, f and g are independently 0 or 1; 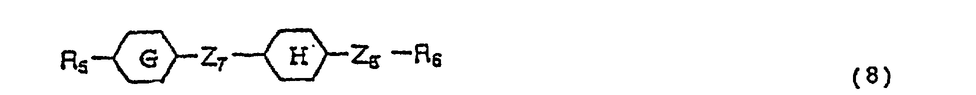 wherein R₅ and R₆ are independently a C₁-C₁₀ alkyl group or C₂-C₁₀ alkenyl group, any not adjacent methylene group in said alkyl group or alkenyl group may be replaced by oxygen atom; ring G is trans-1,4-cyclohexylene group, 1,4-phenylene group, or pyrimidine-2,5-diyl group; ring H is trans-1,4-cyclohexylene group or 1,4-phenylene group; Z₇ is -C≡C-, -COO-, -(CH₂)₂-, -CH=CH-C≡C-, or single bond; and Z₈ is -COO-, or single bond; and 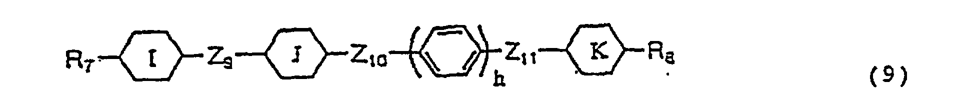 wherein R₇ and R₈ are independently a C₁-C₁₀ alkyl group or C₂-C₁₀ alkenyl group, any not adjacent methylene group in said alkyl group or alkenyl group may be replaced by oxygen atom; ring I is trans-1,4-cyclohexylene group, 1,4-phenylene group, or pyrimidine-2,5-diyl group; ring J is trans-1,4-cyclohexylene group, 1,4-phenylene group one or more hydrogen atoms on the ring may be replaced by F, or pyrimidine-2,5-diyl group; ring K is a trans-1,4-cyclo-hexylene group or a 1,4-phenylene group; Z₉ and Z₁₁ are independently -COO-, -(CH₂)₂-, or single bond; Z₁₀ is -CH=CH-, -C≡C-, -COO-, or single bond; and h is 0 or 1.

7. A liquid crystal composition according to claim 4, further comprising, as a part of a second component, at least one compound selected from the group consisting of the compounds expressed by any one of the general formulae (2), (3) and (4) as defined in claim 5, and comprising, as another part of the second component, at least one compound selected from the group consisting of the compounds expressed by any one of the general formulae (5), (6), (7), (8), and (9) as defined in claim 6.

8. A liquid crystal display device including a liquid crystal composition comprising at least two components and comprising at least one liquid crystalline compound defined in any one of claims 1 to 3.

9. A liquid crystal display device according to claim 8 including a liquid crystal composition as defined in claims 5,6 or 7.

## Patentansprüche

1. Eine flüssigkristalline Verbindung der allgemeinen Formel (1): 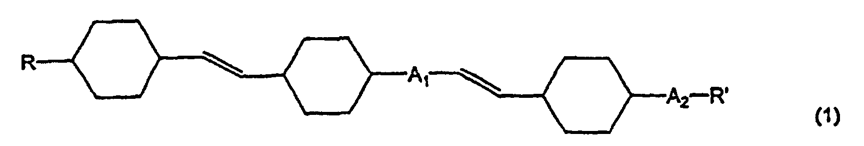 worin R eine C₁₋₁₀-Alkyl-Gruppe oder C₂₋₁₀-Alkenyl-Gruppe ist; R' eine C₁₋₁₀-Alkyl-Gruppe, eine C₂₋₁₀-Alkenyl-Gruppe, F, Cl, CN, OCF₃, CF₃, OCF₂H, CF₂H oder CFH₂ ist; A₁ und A₂ unabhängig voneinander eine trans-1,4-Cyclohexylen-Gruppe, 1,4-Phenylen-Gruppe, in der ein oder mehrere Wasserstoffatome am 6-gliedrigen Ring durch ein Halogenatom ersetzt werden können, oder eine Einfachbindung ist, unter der Voraussetzung, daß A₁ und A₂ so ausgewählt werden, daß die Zahl der Ringe in der Verbindung der Formel (1) vier ist; und jedes der die Verbindung aufbauenden Elemente durch ein Isotop ersetzt werden kann.

2. Flüssigkristalline Verbindung gemäß Anspruch 1, worin in der allgemeinen Formel (1) A₁ eine trans-1,4-Cyclohexylen-Gruppe ist und A₂ eine Einfachbindung ist.

3. Flüssigkristalline Verbindung gemäß Anspruch 1, worin in der allgemeinen Formel (1) A₁ eine Einfachbindung und A₂ eine trans-1,4-Cyclohexylen-Gruppe oder 1,4-Phenylen-Gruppe ist.

4. Flüssigkristallzusammensetzung, die mindestens zwei Bestandteile umfaßt, und mindestens eine flüssigkristalline Verbindung umfaßt, wie sie in einem der Ansprüche 1 bis 3 definiert ist.

5. Flüssigkristallzusammensetzung gemäß Anspruch 4, die ferner als zweiten Bestandteil mindestens eine Verbindung umfaßt, die aus einer Gruppe ausgewählt wird, die aus Verbindungen mit einer der allgemeinen Formeln (2), (3) und (4) besteht: 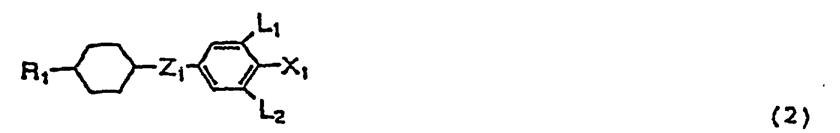 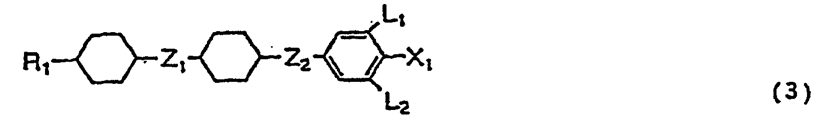 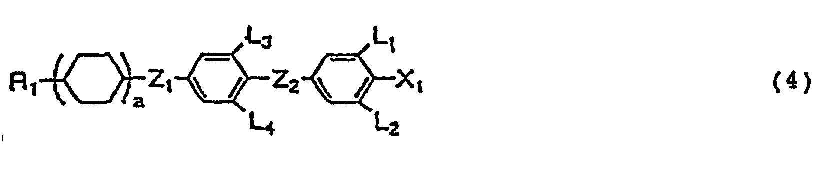 worin R₁ eine C₁₋₁₀-Alkyl-Gruppe ist; X₁ F, Cl, OCF₃, OCF₂H, CF₃, CF₂H oder CFH₂ ist; L₁, L₂, L₃ und L₄ unabhängig voneinander H oder F sind; Z₁ und Z₂ unabhängig voneinander -(CH₂)₂-, -CH=CH- oder eine Einfachbindung sind; und a 1 oder 2 ist.

6. Flüssigkristallzusammensetzung gemäß Anspruch 4, die ferner als zweiten Bestandteil mindestens eine Verbindung umfaßt, die aus einer Gruppe ausgewählt wird, die aus Verbindungen mit einer der allgemeinen Formeln (5), (6), (7), (8) und (9) besteht: 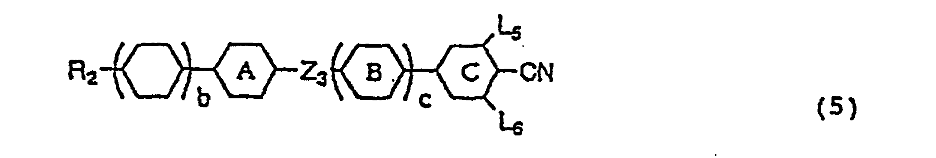 worin R₂ F, eine C₁₋₁₀-Alkyl-Gruppe oder C₂₋₁₀-Alkenyl-Gruppe ist, wobei eine oder mehrere nicht benachbarte Methylen-Gruppe(n) in der Alkyl-Gruppe oder Alkenyl-Gruppe durch ein Sauerstoffatom ersetzt werden können; Ring A eine trans-1,4-Cyclohexylen-Gruppe, 1,4-Phenylen-Gruppe, Pyrimidin-2,5-diyl-Gruppe oder 1,3-Dioxan-2,5-diyl-Gruppe ist; Ring B eine trans-1,4-Cyclohexylen-Gruppe, 1,4-Phenylen-Gruppe oder Pyrimidin-2,5-diyl-Gruppe ist; Ring C eine trans-1,4-Cyclohexylen-Gruppe oder 1,4-Phenylen-Gruppe ist; Z₃ eine -(CH₂)₂-, -COO- oder eine Einfachbindung ist; L₅ und L₆ unabhängig voneinander H oder F sind; und b und c unabhängig voneinander 0 oder 1 sind; 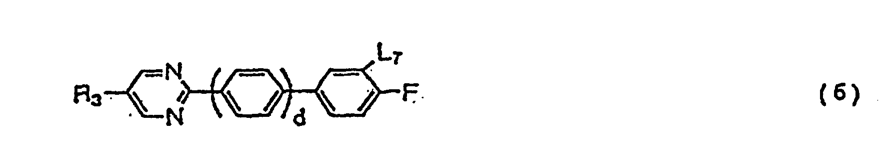 worin R₃ eine C₁₋₁₀-Alkyl-Gruppe ist; L₇ H oder F ist; und d 0 oder 1 ist; 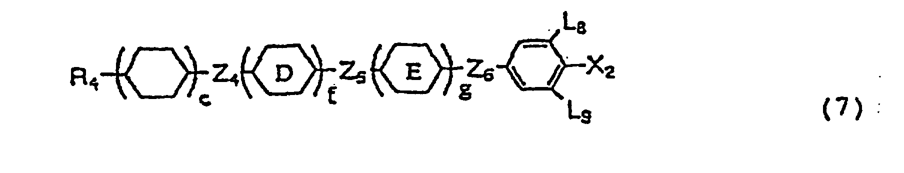 worin R₄ eine C₁₋₁₀-Alkyl-Gruppe ist; die Ringe D und E unabhängig voneinander eine trans-1,4-Cyclohexylen-Gruppe oder 1,4-Phenylen-Gruppe sind; Z₄ und Z₅ unabhängig voneinander -COO- oder eine Einfachbindung sind; Z₆ -COO- oder -C≡C- ist; L₈ und L₉ unabhängig voneinander H oder F sind; X₂ F, OCF₃, OCF₂H, CF₃, CF₂H oder CFH₂ ist; und e, f und g unabhängig voneinander 0 oder 1 sind; 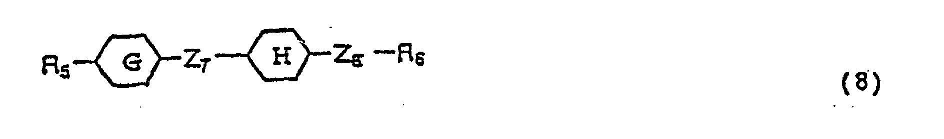 worin R₅ und R₆ unabhängig voneinander eine C₁₋₁₀-Alkyl-Gruppe oder C₂₋₁₀-Alkenyl-Gruppe sind, wobei eine oder mehrere nicht benachbarte Methylen-Gruppe(n) in der Alkyl-Gruppe oder Alkenyl-Gruppe durch ein Sauerstoffatom ersetzt werden können; Ring G eine trans-1,4-Cyclohexylen-Gruppe, 1,4-Phenylen-Gruppe oder Pyrimidin-2,5-diyl-Gruppe ist; Ring H eine trans-1,4-Cyclohexylen-Gruppe oder 1,4-Phenylen-Gruppe ist; Z₇ -C≡C-, -COO-, -(CH₂)₂-, -CH=CH-C≡C- oder eine Einfachbindung ist; und Z₈ -COO- oder eine Einfachbindung ist; und 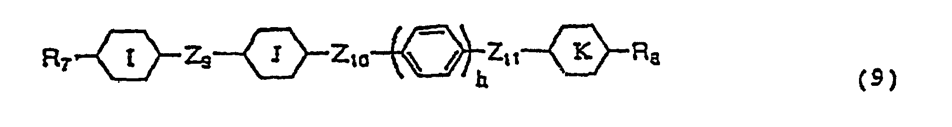 worin R₇ und R₈ unabhängig voneinander eine C₁₋₁₀-Alkyl-Gruppe oder C₂₋₁₀-Alkenyl-Gruppe sind, wobei eine oder mehrere nicht benachbarte Methylen-Gruppe(n) in der Alkyl-Gruppe oder Alkenyl-Gruppe durch ein Sauerstoffatom ersetzt werden können; Ring I eine trans-1,4-Cyclohexylen-Gruppe, 1,4-Phenylen-Gruppe oder Pyrimidin-2,5-diyl-Gruppe ist; Ring J eine trans-1,4-Cyclohexylen-Gruppe, 1,4-Phenylen-Gruppe, wobei ein oder mehrere Wasserstoffatome am Ring durch F ersetzt werden können, oder eine Pyrimidin-2,5-diyl-Gruppe ist; Ring K eine trans-1,4-Cyclohexylen-Gruppe oder eine 1,4-Phenylen-Gruppe ist; Z₉ und Z₁₁ unabhängig voneinander -COO-, -(CH₂)₂- oder eine Einfachbindung sind; Z₁₀ -CH=CH-, -C≡C-, -COO- oder eine Einfachbindung ist; und h 0 oder 1 ist.

7. Flüssigkristallzusammensetzung gemäß Anspruch 4, die ferner als Teil des zweiten Bestandteils mindestens eine Verbindung umfaßt, die aus der Gruppe ausgewählt wird, die aus den Verbindungen mit einer der allgemeinen Formeln (2), (3) und (4) besteht, wie sie in Anspruch 5 definiert sind und als weiteren Teil des zweiten Bestandteils mindestens eine Verbindung umfaßt, die aus der Gruppe ausgewählt wird, die aus Verbindungen mit einer der allgemeinen Formeln (5), (6), (7), (8) und (9) besteht, wie sie in Anspruch 6 definiert sind.

8. Flüssigkristall-Anzeigevorrichtung, die eine Flüssigkristallzusammensetzung enthält, die mindestens zwei Bestandteile umfaßt, und mindestens eine flüssigkristalline Verbindung umfaßt, wie sie in einem der Ansprüche 1 bis 3 definiert ist.

9. Flüssigkristall-Anzeigevorrichtung gemäß Anspruch 8, die eine Flüssigkristallzusammensetzung enthält, wie sie in einem der Ansprüche 5, 6 oder 7 definiert ist.

## Revendications

1. Composé cristallin liquide exprimé par la formule générale (1) : 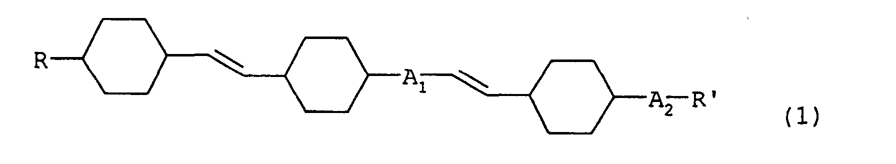 où R est un groupe alkyle en C₁-C₁₀ ou un groupe alcényle en C₂-C₁₀ ; R' est un groupe alkyle en C₁-C₁₀, un groupe alcényle en C₂-C₁₀, F, Cl, CN, OCF₃, CF₃, OCF₂H, CF₂H ou CFH₂ ; A₁ et A₂ sont indépendamment un groupe trans-1,4-cyclohexylène, un groupe 1,4-phénylène où un ou plusieurs atomes d'hydrogène sur le cycle à six chaînons peuvent être remplacés par un atome d'halogène, ou une simple liaison, à condition que A₁ et A₂ soient choisis de manière que le nombre de cycles dans le composé de formule (1) soit quatre ; et chacun des éléments constituant le composé peut être remplacé par un isotope.

2. Composé cristallin liquide selon la revendication 1 où A₁ est un groupe trans-1,4-cyclohexylène et A₂ est une simple liaison dans la formule générale (1).

3. Composé cristallin liquide selon la revendication 1 où A₁ est une simple liaison et A₂ est un groupe trans-1,4-cyclohexylène ou un groupe 1,4-phénylène dans la formule générale (1).

4. Composition de cristaux liquides comprenant au moins deux composants et comprenant au moins un composé cristallin liquide défini dans l'une quelconque des revendications 1 à 3.

5. Composition de cristaux liquides selon la revendication 4 comprenant en outre, comme second composant, au moins un composé choisi dans le groupe consistant en les composés exprimés par l'une des formules générales (2), (3) et (4) : 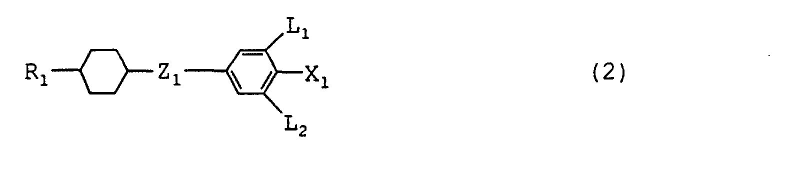 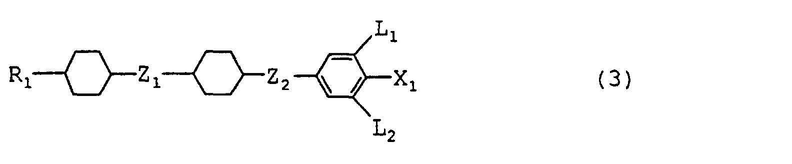 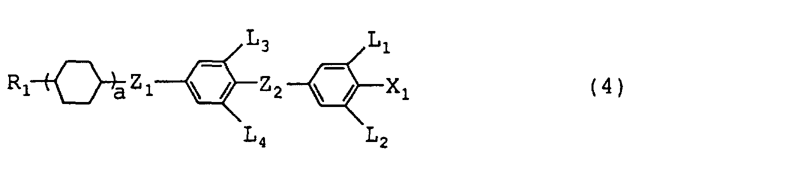 où R₁ est un groupe alkyle en C₁-C₁₀ ; X₁ est F, Cl, OCF₃, OCF₂H, CF₃, CF₂H ou CFH₂ ; L₁, L₂, L₃ et L₄ sont indépendamment H ou F ; Z₁ et Z₂ sont indépendamment -(CH₂)₂-, -CH=CH= ou une simple liaison ; et a est 1 ou 2.

6. Composition de cristaux liquides selon la revendication 4 comprenant en outre, comme second composant, au moins un composé choisi dans le groupe consistant en les composés exprimés par l'une quelconque des formules générales (5), (6), (7), (8) et (9) : 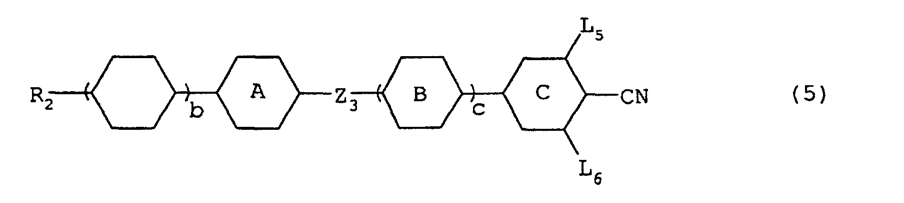 où R₂ est F, un groupe alkyle en C₁-C₁₀ ou un groupe alcényle en C₂-C₁₀, tout groupe méthylène non adjacent dans ledit groupe alkyle ou groupe alcényle peut être remplacé par un atome d'oxygène ; le cycle A est un groupe trans-1,4-cyclohexylène, un groupe 1,4-phénylène, un groupe pyrimidine-2,5-diyle ou un groupe 1,3-dioxane-2,5-diyle ; le cycle B est un groupe trans-1,4-cyclohexylène, un groupe 1,4-phénylène ou un groupe pyrimidine-2,5-diyle ; le cycle C est un groupe trans-1,4-cyclohexylène ou un groupe 1,4-phénylène ; Z₃ est -(CH₂)₂-, -COO- ou une simple liaison ; L₅ et L₆ sont indépendamment H ou F ; et b et c sont indépendamment 0 ou 1 ; 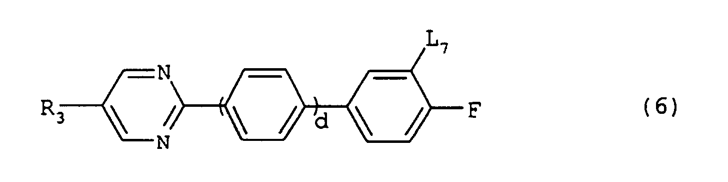 où R₃ est un groupe alkyle en C₁-C₁₀ ; L₇ est H ou F ; et d est 0 ou 1 ; 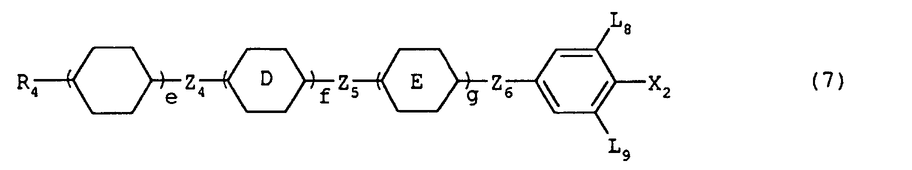 où R₄ est un groupe alkyle en C₁-C₁₀ ; les cycles D et E sont indépendamment un groupe trans-1,4-cyclohexylène ou un groupe 1,4-phénylène ; Z₄ et Z₅ sont indépendamment -COO- ou une simple liaison ; Z₆ est -COO- ou -C≡C- ; L₈ et L₉ sont indépendamment H ou F ; X₂ est F, OCF₃, OCF₂H, CF₃, CF₂H ou CFH₂ ; et e, f et g sont indépendamment 0 ou 1 ; 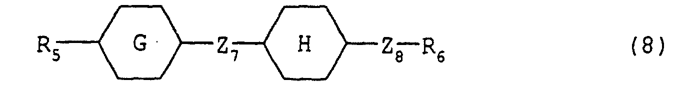 où R₅ et R₆ sont indépendamment un groupe alkyle en C₁-C₁₀ ou un groupe alcényle en C₂-C₁₀, tout groupe méthylène non adjacent dans ledit groupe alkyle ou groupe alcényle peut être remplacé par un atome d'oxygène ; le cycle G est un groupe trans-1,4-cyclohexylène, un groupe 1,4-phénylène ou un groupe pyrimidine-2,5-diyle; le cycle H est un groupe trans-1,4-cyclohexylène ou un groupe 1,4-phénylène ; Z₇ est -C≡C-, -COO-, -(CH₂)₂-, -CH=CH-C≡C- ou une simple liaison ; et Z₈ est -COO- ou une simple liaison ; et 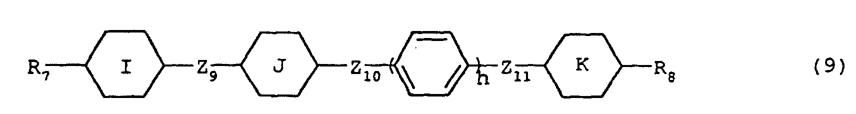 où R₇ et R₈ sont indépendamment un groupe alkyle en C₁-C₁₀ ou un groupe alcényle en C₂-C₁₀, tout groupe méthylène non adjacent dans ledit groupe alkyle ou groupe alcényle peut être remplacé par un atome d'oxygène ; le cycle I est un groupe trans-1,4-cyclohexylène, un groupe 1,4-phénylène ou un groupe pyrimidine-2,5-diyle ; le cycle J est un groupe trans-1,4-cyclohexylène, un groupe 1,4-phénylène, un ou plusieurs atomes d'hydrogène sur le cycle peuvent être remplacés par F, ou un groupe pyrimidine-2,5-diyle; le cycle K est un groupe trans-1,4-cyclohexylène ou un groupe 1,4-phénylène ; Z₉ et Z₁₁ sont indépendamment -COO-, -(CH₂)₂- ou une simple liaison ; Z₁₀ est -CH=CH-, -C≡C-, -COO- ou une simple liaison ; et h est 0 ou 1.

7. composition de cristaux liquides selon la revendication 4 comprenant en outre, comme partie d'un second composant, au moins un composé choisi dans le groupe consistant en les composés exprimés par l'une quelconque des formules générales (2), (3) et (4) définies dans la revendication 5 et comprenant, comme autre partie du second composant, au moins un composé choisi dans le groupe consistant en les composés exprimés par l'une quelconque des formules générales (5), (6), (7), (8) et (9) définies dans la revendication 6.

8. Dispositif d'affichage à cristaux liquides incluant une composition de cristaux liquides comprenant au moins deux composants et comprenant au moins un composé cristallin liquide défini dans l'une quelconque des revendications 1 à 3.

9. Dispositif d'affichage à cristaux liquides selon la revendication 8 incluant une composition de cristaux liquides définie dans les revendications 5, 6 ou 7.
